# EUROPEAN PATENT APPLICATION

(11) **EP 1 230 919 A2**
(43) Date of publication of application: **14.08.2002**
(21) Application number: 02002611.8
(22) Date of filing: 05.02.2002
(51) Int. Cl.: A61K 31/07, A61K 31/517, A61K 31/519, A61P 17/00

(54) **Use of a composition comprising a retinoid and an erb inhibitor in the preparation of a medicament for the treatment of retinoid skin damage**

(30) Priority: 12.02.2001 US 268220 P
(71) Applicant: WARNER-LAMBERT COMPANY, Morris Plains New Jersey 07950 (US)
(72) Inventor: Elder, James Tilford, Ann Arbor, Michigan 48103 (US); Varani, James, Ann Arbor, Michigan 48103 (US)
(74) Representative: Mansmann, Ivo

(57) **Abstract**

Erb inhibitors used in combination with retinoids are effective to prevent skin injury otherwise caused by retinoids alone. A method of treating skin aging and similar skin disorders comprises administering retinoids in combination with erb inhibitors of the general formula 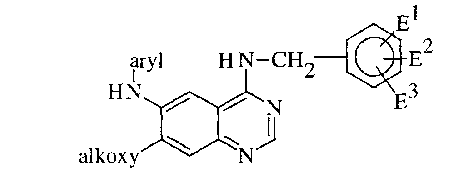 where E¹, E², and E³ include halo, aryl is an alkylcarbonyl or alkenylcarbonyl, and alkoxy is lower alkoxy optionally substituted with amino groups.

## Description

### FIELD OF THE INVENTION

This invention concerns a method for treating skin disease using a combination of retinoids and erb inhibitors. The use of the agents together prevents skin injury caused by retinoids.

### BACKGROUND OF THE INVENTION

In a lifetime, one can expect to experience a wide variety of skin disorders. Skin is an indicator to the health, aging, and disease state of an individual. Afflictions of the skin can be a significant detriment to the health of an individual. Manifestation of diseases of various etiologies (hormonal, metabolic, nutrition, autoimmune, malignancies) often present symptoms cutaneously (Bergfeld W.F., A lifetime of healthy skin: implications for women. *Int J Fertil,* 1999;44(2):83-95). Many skin disorders predispose patients to develop skin cancers.

Effective treatments available to aid in the management of skin disorders are necessary for a human to maintain healthy skin, good physical health, mental health, and a good quality of life. This is especially critical to the aging population, as skin disorders are prevalent with skin aging.

Retinoids are a class of compounds known to be useful in treating disorders of the skin. Numerous compounds defined as retinoids are known (see United States Patent No. 5,49,611), and several are currently used clinically to treat a variety of disorders. For example, 13-cis-retinoic acid (available as Accutane®, Roche Pharmaceuticals) is used to treat skin disorders such as severe recalcitrant nodular acne. Retinoic acid is available as Retin-A® (Ortho Dermatological) and is used to treat acne vulgaris.

There is a toxicity profile of significance associated with retinoid treatment of skin disorder. Many disorders responsive to retinoid treatments require application to large areas of skin that have been affected. Some disorders require retinoid treatment for extended periods of time. Oftentimes, the toxicity characteristic of retinoid treatment causes practitioners to discontinue retinoid treatment and opt for less desirable and less effective treatments.

Topical administration of retinoids are associated with adverse reactions such as: skin irritation, dryness, redness, epidermal hyperplasia. For example, in keratinocytes, retinoids induce proliferation, causing epidermal hyperplasia (Lever L., Marks R., Topical retinoid treatment for skin cancer: a review. *Skin Pharmacology [Switzerland],* 1991;4(3):125-131), edema, blistering, crusting, severe local erythema, burning, stinging pruritus, heightened burning susceptibility upon exposure to sunlight, skin peeling, and overall physical discomfort (*Physicians' Desk Reference,* Montvale, NJ: Medical Economics Company, Inc., 2000:2141).

Retinoids can be administered both topically and systemically, although both routes produce undesirable side effects. Systemic administration of retinoids, for example, accutane (13-cis-retinoic acid) are associated with adverse reactions similar to patients taking high doses of vitamin A. These adverse reactions include dry skin, skin fragility, pruritus, epistaxis, dry nose, and dry mouth. Skeletal hyperostosis has been observed as well as other bone abnormalities. A relationship has been established with the administration of systemic retinoids and depression, psychosis, and suicidal ideation, suicide attempts, and suicide. Patients treated with systemic retinoids experience musculoskeletal symptoms (including arthralgia). Patients also experience transient pain in the chest during treatment. Patients also experience rash (including erythema, seborrhea, and eczema) and thinning of the hair. Patients can also experience peeling of palms and soles, skin infections, nonspecific urogenital findings, nonspecific gastrointestinal symptoms, fatigue headache, and increased susceptibility to sunburn.

While retinoids are clinically useful and are widely prescribed, their utility would be significantly enhanced if their toxicity could be eliminated or at least minimized. We have now discovered that use of compounds known as erb inhibitors, together with retinoids, reduces the undesirable side effects of retinoids, without diminishing their beneficial effects.

Erb inhibitors are compounds that inhibit the activity of one or more of the mammalian enzymes known as epidermal growth factor receptor tyrosine kinases EP 0566226 and EP 0607439. Typical erb inhibitors include those described by Olayioye R.M. et al., The ErbB signaling network: receptor heterodimerization in development and cancer, *EMBO Journal,* 2000:19(13):3159-3167, and in Formulas I, II, and III as described below, incorporated herein by reference.

This invention provides a method whereby erb inhibitors can be used to prevent skin injury caused by retinoids. This invention provides a method for preventing or controlling retinoid toxicity, such as skin irritation, by administering an erb inhibitor.

### SUMMARY OF THE INVENTION

A preferred embodiment of this invention provides a method for treating skin disorder using a combination of retinoids and erb inhibitors. The use of the agents together prevents skin injury caused by retinoids.

Another preferred embodiment of this invention provides a combination of a retinoid and an erb inhibitor.

A preferred aspect of this invention provides a method of treating and preventing skin injury caused by retinoids responsive to erb inhibition by administering a combination of a retinoid and an erb inhibitor.

In a preferred embodiment, the invention is a combination of a quinazoline erb inhibitor together with a retinoid.

In another preferred embodiment, the quinazoline erb inhibitor is a compound selected from the following formulas (WO 97/38938).

In a preferred embodiment, of the present invention provides a method for using compounds having the Formula I 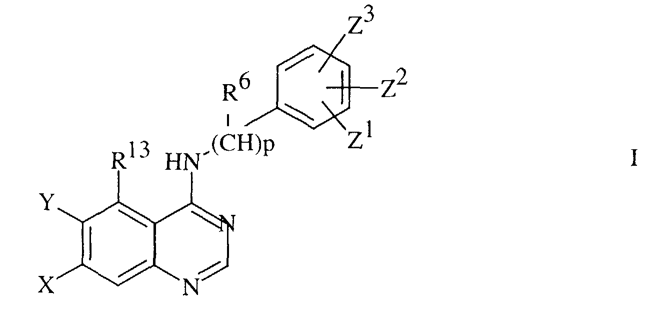 wherein
X is -D-E-F and Y is -SR⁴, halogen, -OR⁴, -NHR³, or hydrogen, or X is -SR⁴, halogen, -OR⁴, -NHR³, or hydrogen, and Y is -D-E-F;
D is 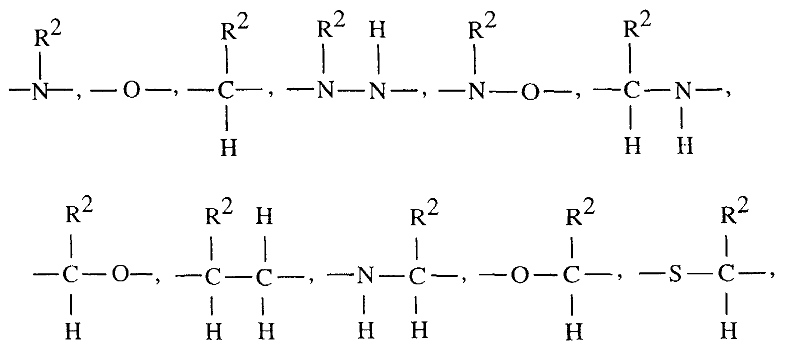 or absent;
E is 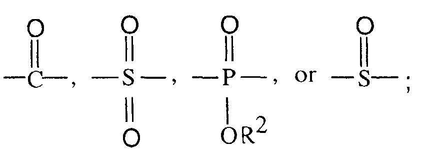
F is 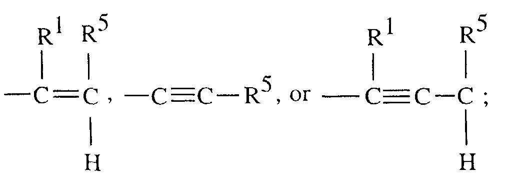
provided that when E is 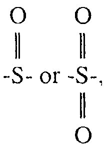
D is not 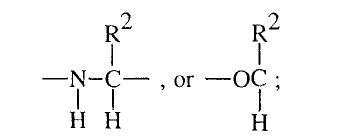
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl,
-(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from -OH, -NH₂, or 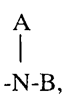 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆-)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
Z¹, Z², or Z³ are independently hydrogen, halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C₆alkyl, -(CH₂)ₙ-N-piperidinyl, -(CH₂)ₙ-piperazinyl, -(CH₂)ₙ-piperazinyl[N₄-(C₁-C₆)alkyl), -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 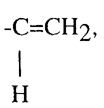 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆alkyl)₂, -1-oxo(C₁-C₆)alkyl, carboxy, (C₁-C₆)alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic
heteroaryl group, and each C₁-C₆ alkyl group above in R⁵ can be substituted with -OH, -NH₂ or -NAB, where A and B are as defined above, R⁶ is hydrogen or C₁-C₆ alkyl; R¹³ is hydrogen or halogen; and
n is 1 to 4, p is 0 or 1, and the pharmaceutically acceptable salts, esters, amides, and prodrugs thereof.

In a preferred embodiment of the compound of Formula I, Z¹ and Z² are hydrogen, and Z³ is a halogen.

In a more preferred embodiment of the compounds of Formula I, Z³ is bromine.

In another more preferred embodiment of the compounds of Formula I, the bromine is located at the 3 or meta position of the phenyl ring.

In another preferred embodiment, Z¹ is hydrogen, Z² is F, and Z³ is Cl.

In another more preferred embodiment, Z¹ is hydrogen, Z² is F, and Z³ is Cl, wherein Z² is located at the 4 position, and Z³ is located at the 3 position of the phenyl ring.

In another preferred embodiment of the compounds of Formula I, X is 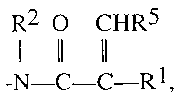 and Y is hydrogen, or
X is hydrogen, and Y is

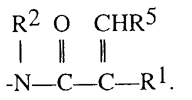

In another preferred embodiment of the compounds of Formula I, Y is -D-E-F, and -D-E-F is 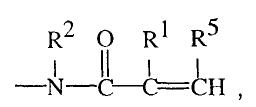 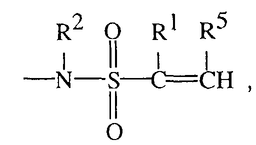 or

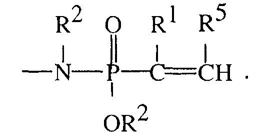

In another preferred embodiment of the compounds of Formula I, X is -D-E-F, and -D-E-F is 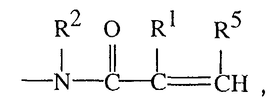 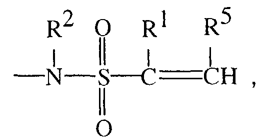 or

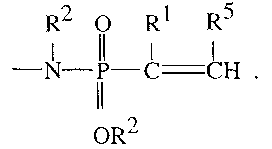

In another preferred embodiment of the compounds of Formula I, R² is hydrogen.

In another preferred embodiment of the compounds of Formula I, Y is -D-E-F and X is -O(CH₂)ₙ-morpholino.

In another preferred embodiment of the compounds of Formula I, R⁵ is carboxy, (C₁-C₆ alkyl)oxycarbonyl or C₁-C₆ alkyl.

In another preferred embodiment of the compounds of Formula I, Y is -D-E-F and X is -O(CH₂)ₙmorpholino.

In another preferred embodiment of the compounds of Formula I, Y is -D-E-F and X is -O-(CH₂)ₙ-N₁-piperazinyl[N₄-(C₁-C₆)alkyl].

In another preferred embodiment of the compounds of Formula I, Y is -D-E-F and X is -O-(CH₂)ₙ-imidazoyl, in combination with a retinoid.

In another embodiment, the present invention provides combinations of retinoids and compounds having the Formula II 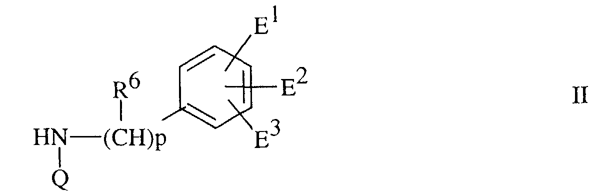 wherein
Q is 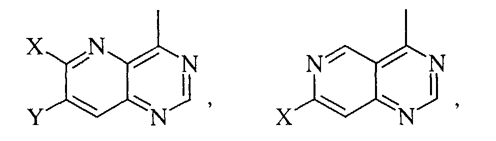 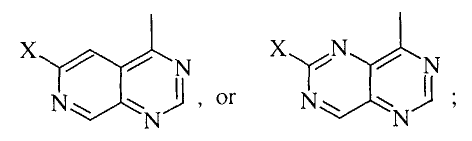
p is 0 or 1;
X is -D-E-F and Y is -SR⁴, -OR⁴, -NHR³, or hydrogen, or X is -SR⁴, -OR⁴, -NHR³, or hydrogen, and Y is -D-E-F;
D is 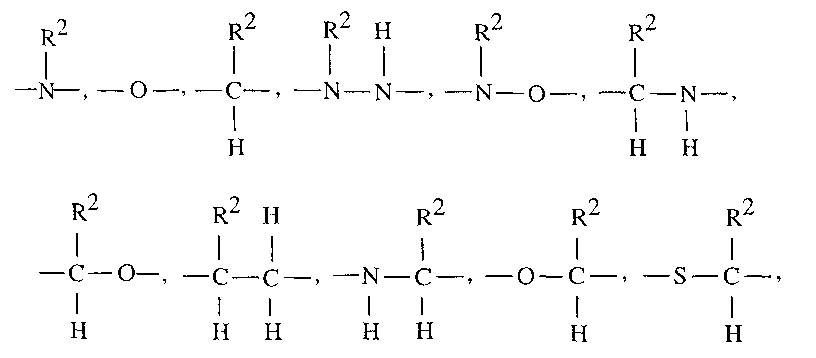 or absent;
E is 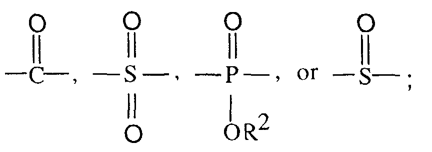
F is 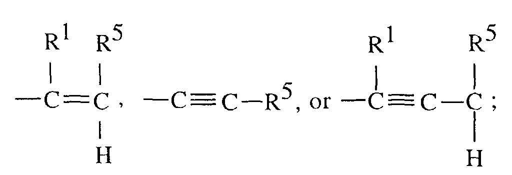 provided that when
E is 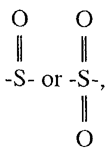
D is not 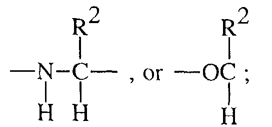
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomolpholino, -(CH₂)ₙ-N-hexahydroazepine or substituted C₁-C₆ alkyl, wherein the substituents are selected from -OH, -NH₂, or 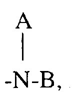 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
E¹, E², and E³ are independently halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C6 alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 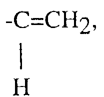 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆ alkyl)₂, -1-oxo(C₁-C₆)alkyl, carboxy, (C₁-C₆) alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group can be substituted with -OH, -NH₂ or -NAB, where A and B are as defined above, R⁶ is hydrogen or C₁-C₆ alkyl; and
n is 1 to 4, p is 0 and 1, and the pharmaceutically acceptable salts, esters, amides, and prodrugs thereof.

In a preferred embodiment of the compounds of Formula II, E¹ and E² are hydrogen, and E³ is a halogen.

In a more preferred embodiment of the compounds of Formula II, the halogen is bromine.

In another more preferred embodiment of the compounds of Formula II, the bromine is located at the three or meta position of the phenyl ring.

In another more preferred embodiment, E¹ is hydrogen, E² is chlorine, and E³ is fluorine.

In another preferred embodiment of the compounds of Formula II, Q is

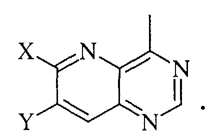

In another preferred embodiment of the compounds of Formula II, Q is

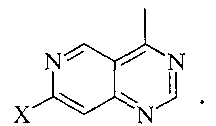

In another preferred embodiment of the compounds of Formula II, Q is

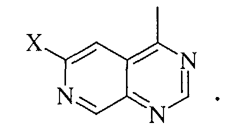

In another preferred embodiment of the compounds of Formula II, Q is

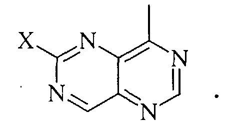

In another preferred embodiment of the compounds of Formula II, X is

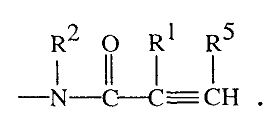

In another preferred embodiment of the compounds of Formula II, X is

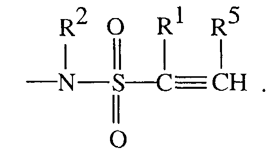

In another preferred embodiment, the present invention provides a method for using compounds having the Formula III 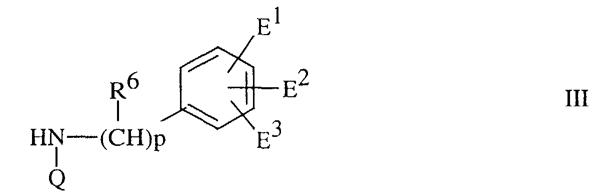 wherein
Q is 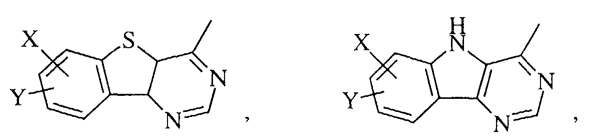 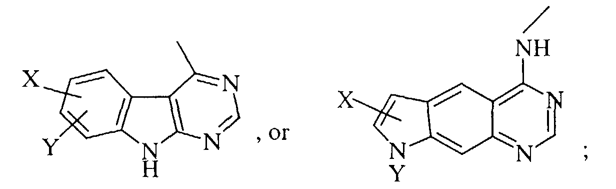
p is 0 or 1;
X is -D-E-F and Y is -SR⁴, -OR⁴, -NHR³, or hydrogen, or X is -SR⁴, -OR⁴, -NHR³, or hydrogen, and Y is -D-E-F;
D is 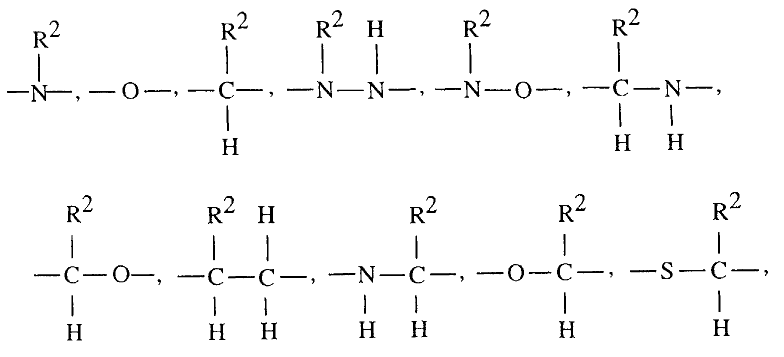 or absent;
E is 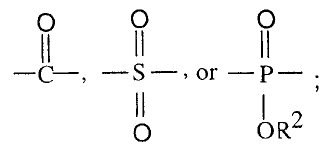
F is 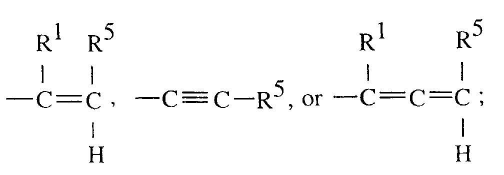 provided that when
E is 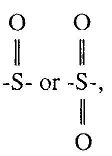
D is not 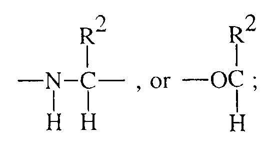
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from -OH, -NH₂, or 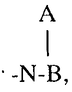 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
E¹, E², and E³ are independently halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;

R⁵ is hydrogen, halogen, C₁-C₆-perfluoroalkyl, 1,1-difluoro(C₁-C₆)alkyl, C₁-C₆ alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 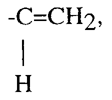 -(CH₂)ₙ-N-hexahydroazepine, -(CH₂)ₙNH₂, -(CH₂)ₙNH(C₁-C₆ alkyl), -(CH₂)ₙN(C₁-C₆ alkyl)₂, -1-oxo(C₁-C₆)alkyl, carboxy, (C₁-C₆)alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group can be substituted with -OH, -NH₂ or -NAB, where A and B are as defined above, R⁶is hydrogen or C₁-C₆ alkyl; and
n is 1 to 4, and the pharmaceutically acceptable salts, esters, amides, and prodrugs thereof in combination with a retinoid.

In another preferred embodiment of the compounds of Formula III, Q is

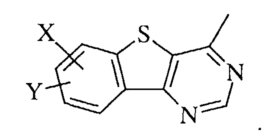

In another preferred embodiment of the compounds of Formula III, Q is

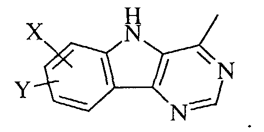

In another preferred embodiment of the compounds of Formula III, X is

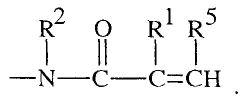

In another preferred embodiment of the compounds of Formula III, E¹ and E² are hydrogen ,and E³ is bromine.

In another preferred embodiment of the compounds of Formula III, E¹ is hydrogen, E² is chlorine, and E³ is fluorine.

In another preferred embodiment of the compounds of Formula III, X is

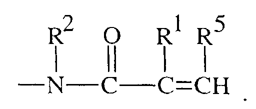

In another preferred embodiment, Q is a 6-substituted benzothieno[3,2-d]-pyrmid-4-yl.

In yet another preferred embodiment, the present invention also provides a pharmaceutically acceptable composition that comprises a compound of Formula I, II, or III.

In another preferred aspect, the present invention also provides a method of treating cancer, the method comprising administering to a patient having cancer a therapeutically effective amount of a compound of Formula I, II, or III.

In a more preferred embodiment, the present invention also provides a method of treating or preventing restenosis, the method comprising administering to a patient having restenosis or at risk of having restenosis, a therapeutically effective amount of a compound of Formula I, II, or III.

In a preferred embodiment, the present invention also provides a method of treating psoriasis, the method comprising administering to a patient having psoriasis a therapeutically effective amount of a compound of Formula I, II, or III.

In a preferred embodiment, the present invention also provides a method of treating atherosclerosis, the method comprising administering to a patient having atherosclerosis a therapeutically effective amount of a compound of Formula I, II, or III.

In yet another preferred embodiment, the present invention also provides a method of treating endometriosis, the method comprising administering to a patient having endometriosis a therapeutically effective amount of a compound of Formula I, II, or III.

In yet another preferred embodiment, the present invention also provides a method of irreversibly inhibiting tyrosine kinases, the method comprising administering to a patient in need of tyrosine kinase inhibition a tyrosine kinase inhibiting amount of a compound of Formula I, II or III.

In a preferred embodiment, the present invention provides combinations of retinoids together with erb inhibitors selected from:
N-[4-(3-Bromo-phenylamino)-pyrido[4,3-d]-pyrimidin-7-yl]-N-(3-morpholin-4-yl-propyl)-acrylamide;
N-[4-(3-Bromo-phenylamino)-pyrido[3,4-d]-pyrimidin-6-yl]-N-(3-morpholin-4-yl-propyl)-acrylamide;
N-[4-(3-Bromo-phenylamino)-quinazolin-7-yl]acrylamide;
N-[4-[(3-Bromophenyl)amino]quinazolin-7-yl]-N-[3-morpholinopropyl]acrylamide;
3-[4-(3-Bromo-phenylamino)-quinazolin-7-yl-carbamoyl]-acrylic acid;
3-[4-(3-Bromo-phenylamino)-quinazolin-7-yl-carbamoyl]-acrylic acid ethyl ester;
But-2-enoic acid [4-(3-bromo-phenylamino)-quinazolin-7-yl]-amide;
N-[4-(3-Bromo-phenylamino)-6-(3-morpholin-4-yl-propylamino)-quinazolin-7-yl]-acrylamide;
N-[4-[(3-Bromophenyl)amino]quinazolin-6-yl]acrylamide;
N-[4-(3-Methyl-phenylamino)-quinazolin-7-yl]acrylamide;
N-[4-(3-Chloro-phenylamino)-quinazolin-7-yl]acrylamide;
N-[4-(3- Bromo-phenylamino)-quinazolin-7-yl]methacrylamide;
N-[4-(3-Bromo-phenylamino)-quinazolin-7-yl]ethenylsulfonamide;
N-[4-[(3-Chlorophenyl)amino]quinazolin-6-yl]acrylamide;
N-[4-[(3-Methylphenyl)amino]quinazolin-6-yl]acrylamide;
N-[4-[(3-(Trifluoromethyl)phenyl)amino]quinazolin-6-yl]acrylamide;
N-[4-[(3-Bromophenyl)amino]-7-[3-(4-morpholino)-propoxy]quinazolin-6-yl]acrylamide;
N-[4-[(3-Methylphenyl)amino]-7-[3-(4-morpholino)propoxy]quinazolin-6-yl]acrylamide;
N-[4-[(3-Methylphenyl)amino]-7-[3-(4,N-methyl-1,N-piperazino)-propoxy]quinazolin-6-yl]acrylamide;
N-[4-[(3-Bromophenyl)amino]-7-[3-(4,N-methyl-1,N-piperazino)-propoxy]quinazolin-6-yl]acrylamide;
N-[4-[(3-Bromophenyl)amino]-7-[3-(1,N-imidazyl)propoxy]quinazolin-6-yl]acrylamide;
N-[4-[(3-Bromophenyl)amino]-7-[4-(N,N-dimethyl-amino)butoxy]-quinazolin-6-yl]acrylamide;
N-[4-[(3-Bromophenyl)amino]quinazolin-6-yl]-N-[3-morpholinopropyl]-acrylamide;
N-[4-[(3-Bromophenyl)amino]quinazolin-6-yl]methacrylamide;
N-[4-(3-Bromo-phenylamino)-quinazolin-7-yl]ethenylsulfonamide;
N-[4-[(3-Bromophenyl)amino]quinazolin-6-yl]-E-but-2-enamide;
N-[4-[(3-Bromophenyl)amino]quinazolin-6-yl]-4,4,4-trifluoro-E-but-2-enamide;
N-[4-[(3-Bromophenyl)amino]quinazolin-6-yl]propynamide;
N-[4-[(3-Bromophenyl)amino]quinazolin-6-yl]but-2-ynamide;
N-[4-(3-Bromo-phenylamino)-pyrido[4,3-d]pyrimidin-7-yl]-acrylamide;
N-[4-(3-Bromo-phenylamino)-pyrido[3,4-d]pyrimidin-6-yl]-acrylamide;
N-[4-(3-Methyl-phenylamino)-pyrido[3,4-d]pyrimidin-6-yl]-acrylamide;
N-[4-(3-Bromo-phenylamino)-pyrido[3,4-d]pyrimidin-6-yl]-N-methyl acrylamide;
N-[4-(3-Bromo-phenylamino)-pyrido[3,4-d]pyrimidin-6-yl]-methacrylamide;
N-[4-(3-Bromo-phenylamino)-pyrido[3,4-d]pyrimidin-6-yl]-ethenylsulfonamide;
N-[4-(3-Bromo-phenylamino)-pyrido[3,2-d]pyrimidin-6-yl]-acrylamide;
N-[4-(3-Bromo-phenylamino)-benzo[b]thieno[3,2-d]pyrimidin-8-yl]acrylamide;
N-[4-(3-Bromo-phenylamino)-benzo[b]thieno[3,2-d]pyrimidin-6-yl]acrylamide;
N-[4-(3-Bromo-phenylamino)-benzo[b]thieno[3,2-d]pyrimidin-7-yl]acrylamide;
N-[4-[(3-Bromophenyl)amino]quinazolin-6-yl]buta-2,3-dienamide;
N-[4-[(3-Bromophenyl)amino]quinazolin-6-yl]-E,4-oxopent-2-enamide;
N-[4-[(3-Bromophenyl)amino]quinazolin-6-yl]-E,4-ethoxy-4-oxobut-2-enamide;
N-[4-(3-Bromo-phenylamino)-pyrido[3,4-d]pyrimidin-6-yl]penta-2,4-dienamide;
N-[4-(3-Bromo-phenylamino)-pyrido[3,4-d]pyrimidin-6-yl]-N-(2-(N,N-dimethylamino)ethyl)acrylamide;
N-[4-(3-Bromo-phenylamino)-pyrido[3,4-d]pyrimidin-6-yl]E-but-2-enamide;
N-[4-(3-Bromo-phenylamino)-pyrido[3,4-d]pyrimidin-6-yl]cinnamide;
N-[4-(3-Bromo-phenylamino)-pyrido[3,4-d]pyrimidin-6-yl]-E,3-chloroacrylamide;
N-[4-(3-Bromo-phenylamino)-pyrido[3,4-d]pyrimidin-6-yl]-propynamide;
N-[4-[(3-Bromophenyl)amino]quinazolin-6-yl]-E,4-(3-(N,N-dimethylamino)propoxy-4-oxobut-2-enamide tris trifluoroacetate;
3-[4-(3-Bromo-phenylamino)-quinazolin-6-ylcarbamoyl]-acrylic acid (Z);
N-[4-[(3-Bromophenyl)amino]quinazolin-6-yl]-E,4-(3-(N,N-dimethylamino)propylamino-4-oxobut-2-enamide;
4-[(3-Bromo-phenyl)amino]-6-(ethenesulfonyl)pyrido[3,4-d]pyrimidine;
1-[4-(3-Bromo-phenylamino)-quinazolin-6-yl]-pyrrole-2,5-dione;
1-[4-(3-Bromo-phenylamino)-quinazolin-6-yl]-prop-2-en-1-one;
Acrylic acid 4-(3-bromo-phenylamino)-quinazolin-6-yl ester;
Methyl N-[4-[(3-bromophenyl)amino]-P-ethenyl-pyrido[3,4-d]pyrimidin-6-yl]phosphonamidate;
Acrylic acid 4-(3-bromo-phenylamino)-quinazolin-7-yl ester;
1-[4-(3-Bromo-phenylamino)-quinazolin-6-yl]-but-3-en-2-one;
Acrylic acid 4-(3-chloro-4-fluoro-phenylamino)-7-methoxy-quinazolin-6-yl ester;
N-[4-(3-Bromo-phenylamino)-7-(3-morpholin-4-yl-propoxy)-pyrido[3,2-d]pyrimidin-6-yl]-acryl amide;
Penta-2,3-dienoic acid [4-(3-bromo-phenylamino)-quinazolin-6-yl]-amide;
Propa-1,2-diene-1-sulfonic acid [4-(3-bromo-phenylamino)-quinazolin-6-yl]-amide;
Methyl N-[4-[(3-bromophenyl)amino]-6-quinazolinyl]-P-(1,2-propadienyl)phosphonamidate;
N-[1-(3-Bromo-phenylamino)-9H-2,4,9-triaza-fluoren-7-yl]-acrylamide;
N-[4-(3-Bromo-phenylamino)-9H-1,3,9-triaza-fluoren-6-yl]-acrylamide;
N-[4-(3-Chloro-4-fluoro-phenylamino)-quinazolin-6-yl]-acrylamide;
N-(4-Phenylmethylamino-quinazolin-6-yl)-acrylamide;
(S)-N-[4-(1-Phenyl-ethylamino)-quinazolin-6-yl]-acrylamide;
(R)-N-[4-(1-Phenyl-ethylamino)-quinazolin-6-yl]-acrylamide;
But-2-enedioic acid [4-(3-chloro-4-fluoro-phenylamino)-quinazolin-6-yl]-amide (3-dimethylamino-propyl)-amide;
N-[4-(3-Chloro-4-fluoro-phenylamino)-pyrido[3,4-d]pyrimidin-6-yl]-acrylamide;
N-[4-(3-Chloro-4-fluoro-phenylamino)-pyrido[3,4-d]pyrimidin-6-yl]-N-methyl-acrylamide;
But-2-enedioic acid [4-(3-chloro-4-fluoro-phenylamino)-pyrido[3,4-d]-pyrimidin-6-yl]-amide (3-dimethylamino-propyl)-amide;
But-2-enedioic acid [4-(3-chloro-4-fluoro-phenylamino)-pyrido[3,4-d]-pyrimidin-6-yl]-amide (3-imidazol-1-yl-propyl)-amide;
4,4-Difluoro-8-morpholin-4-yl-oct-2-enoic acid [4-(3-chloro-4-fluorophenylamino)-pyrido[3,4-d]pyrimidin-6-yl]-amide;
8-Dimethylamino-4,4-difluoro-oct-2-enoic acid [4-(3-chloro-4-fluorophenylamino)-pyrido[3,4-d]pyrimidin-6-yl]-amide;
7-Dimethylamino-4,4-difluoro-hept-2-enoic acid [4-(3-chloro-4-fluorophenylamino)-pyrido[3,4-d]pyrimidin-6-yl]-amide;
4,4-Difluoro-7-morpholin-4-yl-hept-2-enoic acid [4-(3-chloro-4-fluorophenylamino)-pyrido[3,4-d]pyrimidin-6-yl]-amide;
6-Dimethylamino-hex-2-ynoic acid [4-(3-chloro-4-fluoro-phenylamino)-pyrido[3,4-d]pyrimidin-6-yl]-amide;
6-Morpholin-4-yl-hex-2-ynoic acid [4-(3-chloro-4-fluoro-phenylamino)-pyrido[3,4-d]pyrimidin-6-yl]-amide;
7-Dimethylamino-hept-2-ynoic acid [4-(3-chloro-4-fluoro-phenylamino)-pyrido[3,4-d]pyrimidin-6-yl]-amide;
7-Morpholin-4-yl-hept-2-ynoic acid [4-(3-chloro-4-fluoro-phenylamino)-pyrido[3,4-d]pyrimidin-6-yl]-amide;
5-Dimethylamino-pent-2-ynoic acid [4-(3-chloro-4-fluoro-phenylamino)-pyrido[3,4-d]pyrimidin-6-yl]-amide;
5-Morpholin-4-yl-pent-2-ynoic acid [4-(3-chloro-4-fluoro-phenylamino)-pyrido[3,4-d]pyrimidin-6-yl]-amide;
5-Imidazol-1-yl-pent-2-ynoic acid [4-(3-chloro-4-fluoro-phenylamino)-pyrido[3,4-d]pyrimidin-6-yl]-amide;
5-(4-Methyl-piperazin-1-yl-pent-2-ynoic acid [4-(3-chloro-4-fluorophenylamino)-pyrido[3,4-d]pyrimidin-6-yl]-amide;
4-[4-(3-Chloro-4-fluoro-phenylamino)-pyrido[3,4-d]pyrimidin-6-ylcarbamoyl]-but-3-enoic acid 2-(4-methyl-piperazin-1-yl)-ethyl ester;
4-[4-(3-Chloro-4-fluoro-phenylamino)-pyrido[3,4-d]pyrimidin-6-ylcarbamoyl]-but-3-enoic acid 2-(imidazol-1-yl)-ethyl ester;
Pent-2-enedioic acid 1-{[4-(3-chloro-4-fluoro-phenylamino)-pyrido[3,4-d]pyrimidin-6-yl]-amide} 5-[(3-morpholin-4-yl-propyl)-amide];
Pent-2-enedioic acid 1-{[4-(3-chloro-4-fluoro-phenylamino)-pyrido[3,4-d]pyrimidin-6-yl]-amide} 5-[(3-diethylamino-propyl)-amide];
4-[4-(3-Chloro-4-fluoro-phenylamino)-pyrido[3,4-d]pyrimidin-6-ylcarbamoyl]-but-3-enoic acid 2-morpholin-4-yl-ethyl ester;
Pent-2-enedioic acid 1-{[4-(3-chloro-4-fluoro-phenylamino)-pyrido[3,4-d]pyrimidin-6-yl]-amide} 5-{[3-4-methyl-piperazin-1-yl)-propyl]-amide};
(3-Chloro-4-fluoro-phenyl)- {6-[2-(3-dimethylamino-propoxy)-ethenesulfonyl]-pyrido[3,4-d]pyrimidin-4-yl}-amine;
(3-Chloro-4-fluoro-phenyl)-(6- {2-[4-(4-methyl-piperazin-1-yl)-butylamino]-ethenesulfonyl}-pyrido[3,4-d]pyrimidin-4-yl)-amine;
(3-Chloro-4-fluoro-phenyl)-[6-(5-morpholin-4-yl-pent-1-ene-1-sulfonyl)-pyrido[3,4-d]pyrimidin-4-yl]-amine;
(3-Chloro-4-fluoro-phenyl)-(6-ethenesulfinyl-pyrido[3,4-d]pyrimidin-4-yl]-amine;
3-[4-(1-Phenyl-ethylamino)-quinazolin-6-ylcarbamoyl]-acrylic acid 2-morpholin-4-yl-ethyl ester;
But-2-enedioic acid (4-imidazol-1-yl-butyl)-amide [4-( 1-phenyl-ethylamino)-quinazolin-6-yl]-amide;
4-[4-(1-Phenyl-ethylamino)-quinazolin-6-ylcarbamoyl]-but-3-enoic acid 3-diethylamino-propyl ester;
Pent-2-enedioic acid 5-{[2-(4-methyl-piperazin-1-yl)-ethyl]-amide} 1-{[4-(1-phenyl-ethylamino)-quinazolin-6-yl]-amide};
4,4-Difluoro-7-morpholin-4-yl-hept-2-enoic acid [4-(1-phenyl-ethylamino)-quinazolin-6-yl]-amide;
7-Dimethylamino-4,4-difluoro-hept-2-enoic acid [4-(1-phenyl-ethylamino)-quinazolin-6-yl]-amide;
7-Imidazol-1-yl-hept-2-ynoic acid [4-(1-phenyl-ethylamino)-quinazolin-6-yl]-amide;
6-Dimethylamino-hex-2-ynoic acid [4-(1-phenyl-ethylamino)-quinazolin-6-yl]-amide;
But-2-enedioic acid [4-(3-bromo-phenylamino)-pyrido[3,4-d]pyrimidin-6-yl]-amide (3-dimethylamino-propyl)-amide;
But-2-enedioic acid [4-(3-bromo-phenylamino)-pyrido[3,4-d]pyrimidin-6-yl]-amide (3-imidazol-1-yl-propyl)-amide;
4,4-Difluoro-8-morpholin-4-yl-oct-2-enoic acid [4-(3-bromophenylamino)-pyrido[3,4-d]pyrimidin-6-yl]-amide;
8-Dimethylamino-4,4-difluoro-oct-2-enoic acid [4-(3-bromophenylamino)-pyrido[3,4-d]pyrimidin-6-yl]-amide;
7-Dimethylamino-4,4-difluoro-hept-2-enoic acid [4-(3-bromophenylamino)-pyrido[3,4-d]pyrimidin-6-yl]-amide;
4,4-Difluoro-7-morpholin-4-yl-hept-2-enoic acid [4-(3-bromophenylamino)-pyrido[3,4-d]pyrimidin-6-yl]-amide;
6-Dimethylamino-hex-2-ynoic acid [4-(3-bromo-phenylamino)-pyrido[3,4-d]pyrimidin-6-yl]-amide;
6-Morpholin-4-yl-hex-2-ynoic acid [4-(3-bromo-phenylamino)-pyrido[3,4-d]pyrimidin-6-yl]-amide;
7-Dimethylamino-hept-2-ynoic acid [4-(3-bromo-phenylamino)-pyrido[3,4-d]pyrimidin-6-yl]-amide;
7-Morpholin-4-yl-hept-2-ynoic acid [4-(3-bromo-phenylamino)-pyrido[3,4-d]pyrimidin-6-yl]-amide;
5-Dimethylamino-pent-2-ynoic acid [4-(3-bromo-phenylamino)-pyrido[3,4-d]pyrimidin-6-yl]-amide;
5-Morpholin-4-yl-pent-2-ynoic acid [4-(3-bromo-phenylamino)-pyrido[3,4-d]pyrimidin-6-yl]-amide;
5-Imidazol-1-yl-pent-2-ynoic acid [4-(3-bromo-phenylamino)-pyrido[3,4-d]pyrimidin-6-yl]-amide;
5-(4-Methyl-piperazin-1-yl)-pent-2-ynoic acid [4-(3-bromophenylamino)-pyrido[3,4-d]pyrimidin-6-yl]-amide;
4-[4-(3-Bromo-phenylamino)-pyrido[3,4-d]pyrimidin-6-ylcarbamoyl]-but-3-enoic acid 2-(4-methyl-piperazin-1-yl)-ethyl ester;
4-[4-(3-Bromo-phenylamino)-pyrido[3,4-d]pyrimidin-6-ylcarbamoyl]-but-3-enoic acid 2-imidazol-1-yl-ethyl ester;
Pent-2-enedioic acid 1-{[4-(3-bromo-phenylamino)-pyrido[3,4-d]-pyrimidin-6-yl]-amide} 5-[(3-morpholin-4-yl-propyl)-amide];
Pent-2-enedioic acid 1-{[4-(3-bromo-phenylamino)-pyrido[3,4-d]-pyrimidin-6-yl]-amide} 5-[(3-diethylamino-propyl)-amide];
4-[4-(3-Bromo-phenylamino)-pyrido[3,4-d]pyrimidin-6-ylcarbamoyl]-but-3-enoic acid 2-morpholin-4-yl-ethyl ester;
Pent-2-enedioic acid 1-{[4-(3-bromo-phenylamino)-pyrido[3,4-d]-pyrimidin-6-yl]-amide} 5-{[3-(4-methyl-piperazin-1-yl)-propyl]-amide};
(3-Bromo-phenyl)- {6-[2-(3-dimethylamino-propoxy)-ethenesulfonyl]-pyrido[3,4-d]pyrimidin-4-yl }-amine;
(3-Bromo-phenyl)-(6- {2-[4-(4-methyl-piperazin-1-yl)-butylamino]-ethenesulfonyl }-pyrido[3,4-d]pyrimidin-4-yl)-amine;
(3-Bromo-phenyl)-[6-(5-morpholin-4-yl-pent- 1-ene-1-sulfonyl)-pyrido[3,4-d]pyrimidin-4-yl]-amine;
(3-Bromo-phenyl)-(6-ethenesulfinyl-pyrido[3,4-d]pyrimidin-4-yl)-amine;
But-2-enedioic acid [4-(3-chloro-4-fluoro-phenylamino)-quinazolin-6-yl]-amide (3-dimethylamino-propyl)-amide;
But-2-enedioic acid [4-(3-chloro-4-fluoro-phenylamino)-quinazolin-6-yl]-amide (3-imidazol-1-yl-propyl)-amide;
4,4-Difluoro-8-morpholin-4-yl-oct-2-enoic acid [4-(3-chloro-4-fluoro-phenylamino)-quinazolin-6-yl]-amide;
8-Dimethylamino-4,4-difluoro-oct-2-enoic acid [4-(3-chloro-4-fluoro-phenylamino)-quinazolin-6-yl]-amide;
7-Dimethylamino-4,4-difluoro-hept-2-enoic acid [4-(3-chloro-4-fluoro-phenylamino)-quinazolin-6-yl]-amide;
4,4-Difluoro-7-morpholin-4-yl-hept-2-enoic acid [4-(3-chloro-4-fluoro-phenylamino)-quinazolin-6-yl]-amide;
6-Dimethylamino-hex-2-ynoic acid [4-(3-chloro-4-fluorophenylamino)-quinazolin-6-yl]-amide;
6-Morpholin-4-yl-hex-2-ynoic acid [4-(3-chloro-4-fluorophenylamino)-quinazolin-6-yl]-amide;
7-Dimethylamino-hept-2-ynoic acid [4-(3-chloro-4-fluorophenylamino)-quinazolin-6-yl]-amide;
7-Morpholin-4-yl-hept-2-ynoic acid [4-(3-chloro-4-fluorophenylamino)-quinazolin-6-yl]-amide;
5-Dimethylamino-pent-2-ynoic acid [4-(3-chloro-4-fluorophenylamino)-quinazolin-6-yl]-amide;
5-Morpholin-4-yl-pent-2-ynoic acid [4-(3-chloro-4-fluorophenylamino)-quinazolin-6-yl]-amide;
5-Imidazol-1-yl-pent-2-ynoic acid [4-(3-chloro-4-fluorophenylamino)-quinazolin-6-yl]-amide;
5-(4-Methyl-piperazin-1-yl)-pent-2-ynoic acid [4-(3-chloro-4-fluorophenylamino)-quinazolin-6-yl]-amide;
Pent-2-enedioic acid 1-{[4-(3-chloro-4-fluoro-phenylamino)-quinazolin-6-yl]-amide} 5-[(3-morpholin-4-yl-propyl)-amide];
Pent-2-enedioic acid 1-{[4-(3-chloro-4-fluoro-phenylamino)-quinazolin-6-yl]-amide} 5-[(3-diethylamino-propyl)-amide];
4-[4-(3-Chloro-4-fluoro-phenylamino)-quinazolin-6-ylcarbamoyl]-but-3-enoic acid 2-morpholin-4-yl-ethyl ester;
Pent-2-enedioic acid 1-{[4-(3-chloro-4-fluoro-phenylamino)-quinazolin-6-yl]-amide} 5-{[3-(4-methyl-piperazin-1-yl)-propyl]-amide};
(3-Chloro-4-fluoro-phenyl)- 6-[2-(3-dimethylamino-propoxy)-ethenesulfonyl]-quinazolin-4-yl }-amine;
(3-Chloro-4-fluoro-phenyl)-(6-{2-[4-(4-methyl-piperazin-1-yl)-butylamino]-ethenesulfonyl }-quinazolin-4-yl)-amine;
But-2-enedioic acid [4-(3-bromo-phenylamino)-quinazolin-6-yl]-amide (3-dimethylamino-propyl)-amide;
But-2-enedioic acid [4-(3-bromo-phenylamino)-quinazolin-6-yl]-amide (3-imidazol-1-yl-propyl)-amide;
4,4-Difluoro-8-morpholin-4-yl-oct-2-enoic acid [4-(3-bromophenylamino)-quinazolin-6-yl]-amide;
8-Dimethylamino-4,4-difluoro-oct-2-enoic acid [4-(3-bromophenylamino)-quinazolin-6-yl]-amide;
7-Dimethylamino-4,4-difluoro-hept-2-enoic acid [4-(3-bromophenylamino)-quinazolin-6-yl]-amide;
4,4-Difluoro-7-morpholin-4-yl-hept-2-enoic acid [4-(3-bromophenylamino)-quinazolin-6-yl]-amide;
6-Dimethylamino-hex-2-ynoic acid [4-(3-bromo-phenylamino)-quinazolin-6-yl]-amide;
6-Morpholin-4-yl-hex-2-ynoic acid [4-(3-bromo-phenylamino)-quinazolin-6-yl]-amide;
7-Dimethylamino-hept-2-ynoic acid [4-(3-bromo-phenylamino)-quinazolin-6-yl]-amide;
7-Morpholin-4-yl-hept-2-ynoic acid [4-(3-bromophenylamino)-quinazolin-6-yl]-amide;
5-Dimethylamino-pent-2-ynoic acid [4-(3-bromo-phenylamino)-quinazolin-6-yl]-amide;
5-Morpholin-4-yl-pent-2-ynoic acid [4-(3-bromo-phenylamino)-quinazolin-6-yl]-amide;
5-Imidazol-1-yl-pent-2-ynoic acid [4-(3-bromo-phenylamino)-quinazolin-6-yl]-amide;
5-(4-Methyl-piperazin-1-yl)-pent-2-ynoic acid [4-(3-bromophenylamino)-quinazolin-6-yl]-amide;
4-[4-(3-Bromo-phenylamino)-quinazolin-6-ylcarbamoyl]-but-3-enoic acid 2-(4-methyl-piperazin-1-yl)-ethyl ester;
4-[4-(3-Bromo-phenylamino)-quinazolin-6-ylcarbamoyl]-but-3-enoic acid 2-imidazol-1-yl-ethyl ester;
Pent-2-enedioic acid 1-{[4-(3-bromo-phenylamino)-quinazolin-6-yl]-amide 5-[(3-morpholin-4-yl-propyl)-amide];
Pent-2-enedioic acid 1-{[4-(3-bromo-phenylamino)-quinazolin-6-yl]-amide} 5-[(3-diethylamino-propyl)-amide];
4-[4-(3-Bromo-phenylamino)-quinazolin-6-ylcarbamoyl]-but-3-enoic acid 2-morpholin-4-yl-ethyl ester;
Pent-2-enedioic acid 1-{[4-(3-bromo-phenylamino)-quinazolin-6-yl]-amide} 5-{[3-(4-methyl-piperazin-1-yl)-propyl]-amide};
3-[4-(1-Phenyl-ethylamino)-pyrido[3,4-d]pyrimidin-6-ylcarbamoyl]-acrylic acid 2-morpholin-4-yl-ethyl ester;
But-2-enedioic acid (4-imidazol-1-yl-butyl)-amide [4-(1-phenyl-ethylamino)-pyrido[3,4-d]pyrimidin-6-yl]-amide;
4-[4-(1-Phenyl-ethylamino)-pyrido[3,4-d]pyrimidin-6-ylcarbamoyl]-but-3-enoic acid 3-diethylamino-propyl ester;
Pent-2-enedioic acid 5-{[2-(4-methyl-piperazin-1-yl)-ethyl]-amide} 1-{[4-(1-phenyl-ethylamino)-pyrido[3,4-d]pyrimidin-6-yl]-amide};
4,4-Difluoro-7-morpholin-4-yl-hept-2-enoic acid [4-(1-phenyl-ethylamino)-pyrido[3,4-d]pyrimidin-6-yl]-amide;
7-Dimethylamino-4,4-difluoro-hept-2-enoic acid [4-(1-phenyl-ethylamino)-pyrido[3,4-d]pyrimidin-6-yl]-amide;
7-Imidazol-1-yl-hept-2-ynoic acid [4-(1-phenyl-ethylamino)-pyrido[3,4-d]pyrimidin-6-yl]-amide;
6-Dimethylamino-hex-2-ynoic acid [4-(1-phenyl-ethylamino)-pyrido[3,4-d]pyrimidin-6-yl]-amide;
But-2-endioic acid [4-(3-chloro-4-fluorophenylamino)-7-fluoroquinazolin-6-yl]amide (3-dimethylaminopropyl)amide;
But-2-endioic acid [7-chloro-4-(3-chloro-4-fluorophenylamino)quinazolin-6-yl]amide (3-dimethylaminopropyl)amide;
N-[4-[3-(Bromophenyl)amino]-5-fluoro-7-[3-(4-morpholino)propoxy]-quinazolin-6-yl]acrylamide; and
N-[4-[(3-(Chloro-4-fluorophenyl)amino]-5-fluoro-7-(1,N-imidazyl)-propoxy]quinazolin-6-yl]acrylamide.

The most preferred combination is N-[4-(3-chloro-4-fluoro-phenylamino)-7-(3-morpholin-4-yl-propoxy)-quinazolin-6-yl]-acrylamide dihydrochloride together with a retinoid.

Another preferred embodiment is a combination comprising a pyridopyrmidine erb inhibitor together with a retinoid.

Another preferred aspect of this invention provides a combination of an erb inhibitor selected from compounds of Formula I, II, or III.

In another preferred embodiment, this invention provides a combination of 5-(4-methyl-piperazin-1-yl)-pent-2-ynoic acid [4-(3-chloro-4-fluorophenylamino)-pyrido[3,4-d]pyrimidin-6-yl]-amide with a retinoid.

In a preferred embodiment, this invention provides a combination of a quinazoline erb inhibitor together with a retinoid.

In a preferred embodiment, this invention is a combination comprising N-[4-(3-bromo-phenylamino)-7-(3-morpholin-4-yl-propoxy)-quinazolin-6-yl]-acrylamide with a retinoid.

In another preferred embodiment, this invention is a combination of N⁴-(3-bromo-phenyl)-N⁶-methyl-pyrido[3,4-d]pyrimidine-4,6-diamine together with a retinoid.

In another embodiment, the invention provides a combination of N-[4-(3-chloro-4-fluoro-phenylamino)-7-(3-morpholin-4-yl-propoxy)-quinazolin-6-yl]-acrylamide together with a retinoid.

In another preferred embodiment, a method provided by this invention comprises administering an all*-trans* retinal, all*-trans* retinol, all*-trans* retinoic acid, 9-*cis*-retinoic acid, 13-*cis*-retinoic acid, 13-*cis*-retinal, 13-*cis*-retinol, *9-cis*-retinal, or 9-*cis*-retinol in combination with an erb inhibitor to a patient in need of treatment.

In a preferred embodiment of this invention, numerous compounds are known which are characterized as retinoids. A comprehensive discussion of retinoids is given by Dawson and Hobbs, in Chapter 2 of *The Retinoids: Biology, Chemistry, and Medicine,* 2^{nd} ed., Sporn, Roberts, and Goodman. New York: Raven Press, Ltd., 1994. That reference is incorporated herein by reference for its teaching of the synthesis of retinoids. All that is required by this invention is that a compound characterized as a retinoid is administered to an animal in an LF(a) lowering amount.

Preferred retinoids to be utilized in the present invention include retinoic acid of the formula

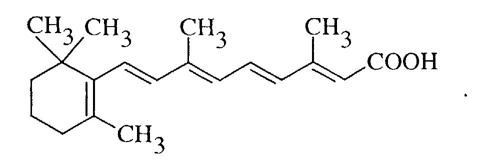

Retinoic acid derivatives also are preferred, for example, compounds of the formula 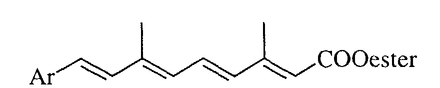 wherein Ar is an aryl group, and "ester" is an organic ester forming group.

Retinoids which are dienyl benzoic acid and enzynylaryl carboxylic acids also are preferred. For example, compounds of the formula 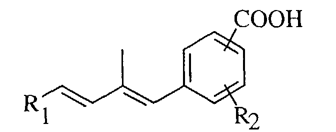 where R₁ is cycloalkyl or aryl, and R₂ is a typical phenyl substituted group such as halo, alkyl, alkoxy, alkylthio, and the like.

Compounds such as 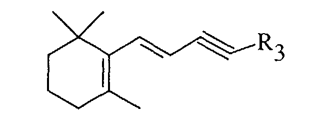 where R₃ is, for instance 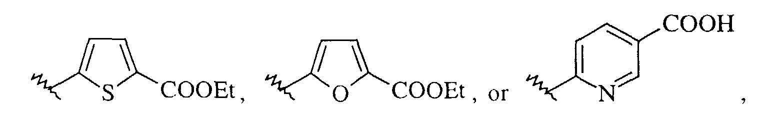 also are preferred.

Preferably, the retinoid is selected from *trans*-retinal, all-*trans* retinol, all-*trans*-retinoic acid, 9-*cis*-retinoic acid, 13-*cis*-retinoic acid, 13-*cis*-retinol, *13-cis*-retinal, 9-*cis*-retinal, or 9-*cis*-retinol.

All of the retinoids required for this invention are known and available by well-known synthetic methodologies.

Preferably, this invention provides a method of treating and preventing skin disorders comprising administering to an animal in need of treatment an effective amount of a combination of an erb inhibitor with a retinoid.

In a further preferred embodiment, this invention provides a method of treating and preventing skin aging comprising administering to an animal in need of treatment an antiaging amount of a combination of a retinoid and an erb inhibitor.

In another preferred embodiment, this invention provides a method of treating skin aging comprising administering a therapeutic amount of a retinoid and an erb inhibitor where the skin aging is photoaging.

In still another preferred embodiment, this invention provides a method of treating and preventing acne comprising administering to an animal in need of treatment an antiacne amount of a combination of an erb inhibitor and a retinoid.

Preferably, this invention provides a method of treating and preventing psoriasis comprising administering to an animal in need of treatment an antipsoriasis amount of a combination of an erb inhibitor and a retinoid.

Preferably, this invention provides a method of treating and preventing precancerous lesions and skin cancers comprising administering to an animal in need of treatment of an anticancer amount of a combination of an erb inhibitor and a retinoid.

In a preferred embodiment, this method also provides a kit containing a retinoid in one compartment and an erb inhibitor in a second compartment.

### DETAILED DESCRIPTION OF THE INVENTION

This invention uses compounds that are known as retinoids. Such retinoids are discussed in Dawson and Hobbs, in Chapter 2 of *The Retinoids: Biology, Chemistry, and Medicine,* 2^{nd} ed., Sporn, Roberts, and Goodman, Raven Press, Ltd, New York, 1994. This reference is incorporated herein by reference. Retinoids include natural forms and synthetic analogues of Vitamin A. Retinoids described in United States Patent No. 5489611 are contemplated to be used in this invention and are incorporated herein by reference. These include but are not limited to all-*trans* retinal, all*-trans* retinol, all*-trans* retinoic acid, 9-cis-retinoic acid, 13-cis-retinal, 13-cis-retinol, 9-cis-retinal, 13-cis-retinoic acid, and 9-cis-retinal, retinol, retinaldehyde, etretinate, and acitretin, aromatic compounds representing second generation compounds, adapine-third generation aromatic retinoid, and newer retinoids.

All that is required to practice this invention is to administer a retinoid in combination with an erb inhibitor. The components can be combined in a single composition, or can be administered individually.

The retinoids are to be administered in doses effective in preventing and treating skin disease. Effective doses of retinoids are described in United States Patent No. 5,489,611 incorporated herein by reference.

Topical retinoids such as RETIN-A®, Renova® (tretinoin emollient cream), and RETIN-A® micro (Ortho Dermatological) are often administered as described in the Physicians' Desk Reference, 2000:2139-2142, incorporated herein by reference.

Gel, Cream, and Liquid, containing tretinoin are used for the topical treatment of acne vulgaris. RETIN-A Gel contains tretinoin (retinoic acid, vitamin A acid) in either of two strengths, 0.025% or 0.01% by weight, in a gel vehicle of butylated hydroxytoluene, hydroxypropyl cellulose and alcohol (denatured with *tert*-butyl alcohol and brucine sulfate) 90% w/w. RETIN-A (tretinoin) Cream contains tretinoin in either of three strengths, 0.1%, 0.05%, or 0.025% by weight, in a hydrophilic cream vehicle of stearic acid, isopropyl myristate, polyoxyl 40 stearate, stearyl alcohol, xanthan gum, sorbic acid, butylated hydroxytoluene, and purified water. RETIN-A Liquid contains tretinoin 0.05% by weight, polyethylene glycol 400, butylated hydroxytoluene and alcohol (denatured with *tert*-butyl alcohol and brucine sulfate) 55%. Chemically, tretinoin is *all-trans*-retinoic acid.

RETIN-A may be applied once to several times a day, enough to cover affected areas of the skin in need of treatment.

For use in the method of this invention, the retinoids preferably are combined with one or more pharmaceutically acceptable diluents, carriers, excipients, or the like, for convenient oral, parenteral, and topical administration to animals, preferably humans. As noted above, the retinoids can be combined with an erb inhibitor and formulated into a single dosage form. The retinoids are ideally suited to formulation for oral administration in the form of tablets, capsules, dispersible powders, granules, suspensions, elixirs, buccal seals, and the like. The formulations typically will contain from about 1% to about 90% by weight of active retinoid, and/or erb inhibitor, and more commonly from about 5% to about 60% by weight.

Oral formulations can contain, for suspensions, from about 0.05% to about 5% by weight of a suspending agent, such as talc or the like, and syrups will contain from about 10% to about 50% by weight of a sugar such as dextrose. Tablets may contain normal amounts of binders, stabilizers, and common diluents in such as corn starch and sugars. Parenteral formulations, for instance, solutions for IV injection, will be made by dissolving or suspending the retinoid in a solvent such as isotonic saline or 5% glucose in sterile water.

The dose of retinoid to be administered is that amount which is effective for treating skin disorders.

An "antiskin disorder amount" of an erb inhibitor is the amount of an erb inhibitor necessary for treating and preventing retinoid-induced skin injury responsive to erb inhibition.

The effective dosage of active ingredients employed may vary depending on the particular compound employed, the mode of administration, and the severity of the condition being treated. However, in general, satisfactory results are obtained when the retinoids are administered at a daily dosage of from about 0.5 to about 500 mg/kg of animal body weight, preferably given in divided doses two to four times a day, or in sustained-release form. For most large mammals, such as humans, the total daily dosage is from about 1 to 100 mg, preferably from about 2 to 80 mg. Dosage forms suitable for internal use comprise from about 0.5 to 500 mg of the active compound in intimate admixture with a solid or liquid pharmaceutically acceptable carrier. This dosage regimen may be adjusted to provide the optimal therapeutic response. For example, several divided doses may be administered daily or the dose may be proportionally reduced as indicated by the exigencies of the therapeutic situation. Typical dosages of erb inhibitor are doses that range from 0.01 mg to 2000 mg; 2 mg to 650 mg; 2 mg, 5 mg, 25 mg, or 250 mg.

The compositions of this invention may be administered orally as well as by intravenous, intramuscular, or subcutaneous routes. Solid carriers include starch, lactose, dicalcium phosphate, microcrystalline cellulose, sucrose, and kaolin, while liquid carriers include sterile water, polyethylene glycols, nonionic surfactants, and edible oils such as corn, peanut, and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT, and BHA.

The preferred pharmaceutical compositions from the standpoint of ease of preparation and administration are solid compositions, particularly tablets and hard-filled or liquid-filled capsules. Oral administration of the compounds is preferred.

These active compounds may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacterial and fungi. The carrier can be a solvent or dispersion medium containing, for example, water, ethanol, polyol (e.g., glycerol, propylene glycol, and liquid polyethylene glycol), suitable mixtures thereof, and vegetable oils.

The compounds may also be encapsulated in liposomes to allow an intravenous administration of the drug. The liposomes suitable for use in the invention are lipid vesicles and may include plurilamellar lipid vesicles, small sonicated multimellar vesicles, reverse phase evaporation vesicles, large multilamellular vesicles, and the like, wherein the lipid vesicles are formed by one or more phospholipids such as phosphotidylcholine, phosphatidylglycerol, sphingomyelin, phospholactic acid, and the like. In addition, the liposomes may also comprise a sterol component such as cholesterol.

In a preferred embodiment, the retinoids and the erb inhibitors are formulated for topical administration, for example as creams, lotions, salves, and in extended release forms such as patches.

The recommended dosage range for Accutane is 0.5 to 2 mg/kg given in two divided doses daily for 15 to 20 weeks. In studies comparing 0.1, 0.5, and 1 mg/kg/day, it was found that all dosages provided initial clearing of disease, but there was a greater need for retreatment with the lower dosages.

It is recommended that for most patients the initial dosage of Accutane be 0.5 to 1 mg/kg/day. Patients whose disease is very severe or is primarily manifested on the body may require up to the maximum recommended dosage, 2 mg/kg/day. During treatment, the dose may be adjusted according to response of the disease and/or the appearance of clinical side effects - some of which may be dose-related.

If the total nodule count has been reduced by more than 70% prior to completing 15 to 20 weeks of treatment, the drug may be discontinued. After a period of 2 months or more off therapy, and if warranted by persistent or recurring severe nodular acne, a second course of therapy may be initiated. Contraceptive measures must be followed for any subsequent course of therapy.

Accutane should be administered with food.

| ACCUTANE DOSING BY BODY WEIGHT | | | | |
|---|---|---|---|---|
| Body Weight | | Total Mg/Day | | |
| kilograms | pounds | 0.5 mg/kg | 1 mg/kg | 2 mg/kg |
| 40 | 88 | 20 | 40 | 80 |
| 50 | 110 | 25 | 50 | 100 |
| 60 | 132 | 30 | 60 | 120 |
| 70 | 154 | 35 | 70 | 140 |
| 80 | 176 | 40 | 80 | 160 |
| 90 | 198 | 45 | 90 | 180 |
| 100 | 220 | 50 | 100 | 200 |

The retinoids may be administered by topical, systemic, intravenous, intramuscular, or subcutaneous routes.

Erb inhibitors belong to the family of receptor tyrosine kinases. Erb receptors are a family of receptors having an extracellular ligand-binding domain, a transmembrane region, and a cytoplasmic protein tyrosine kinase domain. Dimerization of ligands related to EGF bind the extracellular domain of the erb receptors stimulating tyrosine kinase activity which activates autophosphorylation of the tyrosine residues of the cytoplasmic domain. This reaction signals the regulation of intracellular signaling and gene expression biological activity related to erb receptor activation (Olayioye et al., Supra., 2000).

This erbB family consists of the EGF receptor also known as (ERB-B1/HER1), the erbB2/Neu/HER2, erbB3/HER3, and erbB4/HER4 receptor (Olayioye et al., Supra., 2000).

This invention includes the use of reversible and irreversible pharmacological antagonists of tyrosine kinase activity. Such erb inhibitors include but are not limited to the following types of compounds.

Quinazolines and pyridopyrimidines are compounds described in Formulas I, II, and III and RP 0566226, which are incorporated herein by reference.

The invention preferably employs the following erb inhibitors:
N-[4-(3-Chloro-4-fluoro-phenylamino)-7-(3-morpholin-4-yl-propoxy)-quinazolin-6-yl]-acrylamide dihydrochloride 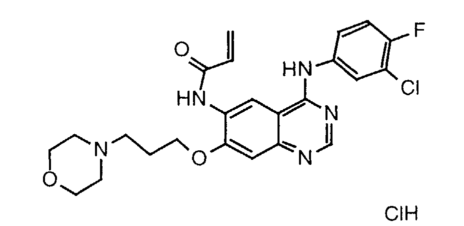 5-(4-Methyl-piperazin-1-yl)-pent-2-ynoic acid [4-(3-chloro-4-fluorophenylamino)-pyrido[3,4-d]pyrimidin-6-yl]-amide 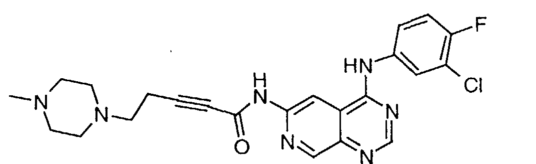
N-[4-(3-Bromo-phenylamino)-7-(3-morpholin-4-yl-propoxy)-quinazolin-6-yl]-acrylamide 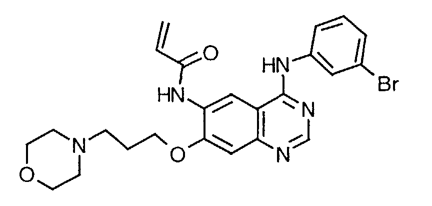
N⁴-(3-Bromo-phenyl)-N⁶-methyl-pyrido[3,4-d]pyrimidine-4,6-diamine 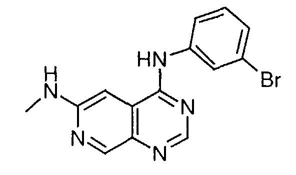
N-[4-(3-Chloro-4-fluoro-phenylamino)-7-(3-morpholin-4-yl-propoxy)-quinazolin-6-yl]-acrylamide

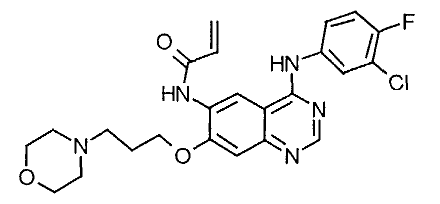

Compositions containing erbB inhibitors, dosage regimens, and administration methods are described in Formulas I, II, and III.

### BRIEF DESCRIPTION OF THE FIGURES

**Figure 1.** Shows the hyperplasia of human skin caused by retinol (ROL) and all*-trans* retinoic acid (RA) after 7 days as measured by the number of keratinocytes per 2 mm of epidermis (block)
**Figure 2.** Shows the hyperplasia to human skin caused by retinoic acid (RA) alone, and the reduction in hyperplasia in the presence of RA together with erb inhibitor PD 169540 (N-[4-(3-bromo-phenylamino)-7-(3-morpholin-4-yl-propoxy)-quinazolin-6-yl]-acrylamide) when measured by the number of keratinocytes/block
**Figure 3a.** Shows the percent survival of human keratinocytes in monolayer culture in the presence of RA (+RA) relative to untreated controls (-RA), and in the presence of the combinations of RA and various concentrations of erb inhibitors PD 169540 (N-[4-(3-bromo-phenylamino)-7-(3-morpholin-4-yl-propoxy)-quinazolin-6-yl]-acrylamide) (0.1, 0.5, and 1 µM) and PD 158780 (N⁴-(3-Bromo-phenyl)-N⁶-methyl-pyrido[3,4-d]pyrimidine-4,6-diamine) (1, 5, and 10 µM)
**Figure 3b.** Shows the percent survival of human keratonocytes in monolayer culture in the absence of RA (-RA), in the presence of RA (+RA) and in the presence of RA plus an antiheperin-binding epidermal growth factor (RA + anti-HB-EGF)
**Figure 4.** Shows the minimal effects on keratinocyte formation caused by retinoic acid (RA) in combination with an antibody to a natural growth factor (anti-HB-EGF)
**Figure 5.** Shows that the ability of erb inhibitors PD 169540 (N-[4-(3-bromo-phenylamino)-7-(3-morpholin-4-yl-propoxy)-quinazolin-6-yl]-acrylamide) and PD 158780 (N⁴-(3-Bromo-phenyl)-N⁶-methyl-pyrido[3,4-d]pyrimidine-4,6-diamine) to antagonize the effects of RA on fibroblast cells is not affected by addition of an antibody to HB-EGF (GF)

The present study examined the effects of RA on human skin subjected to organ culture in the presence or absence of c-erbB antagonists. In parallel, we have examined the effects of these reagents on human epidermal keratinocytes and dermal fibroblasts in monolayer culture. The results strongly support a key role for HB-EGF acting directly on c-erbB family members in keratinocytes in the genesis of RA-induced epidermal hyperplasia.

### MATERIALS AND METHODS

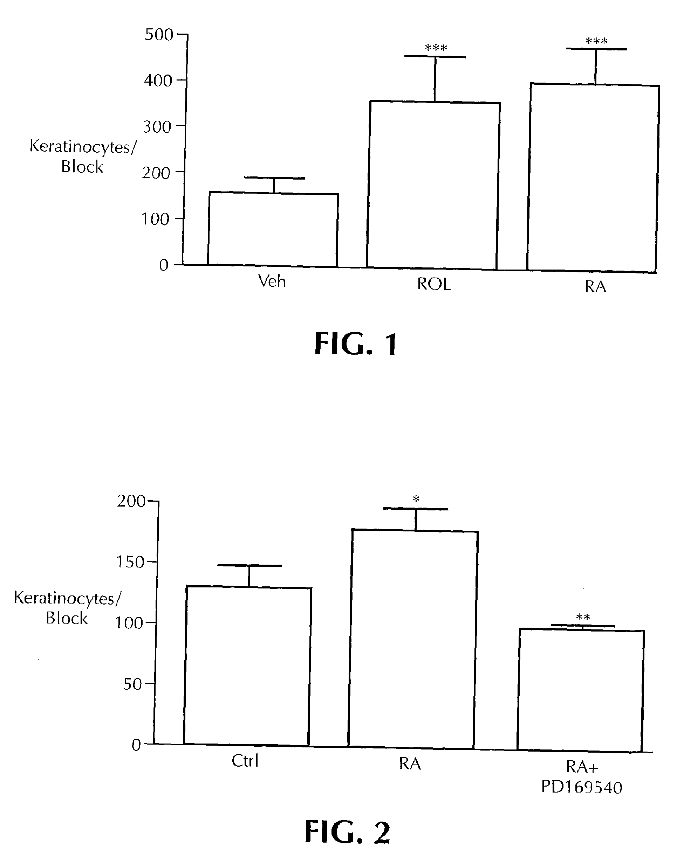

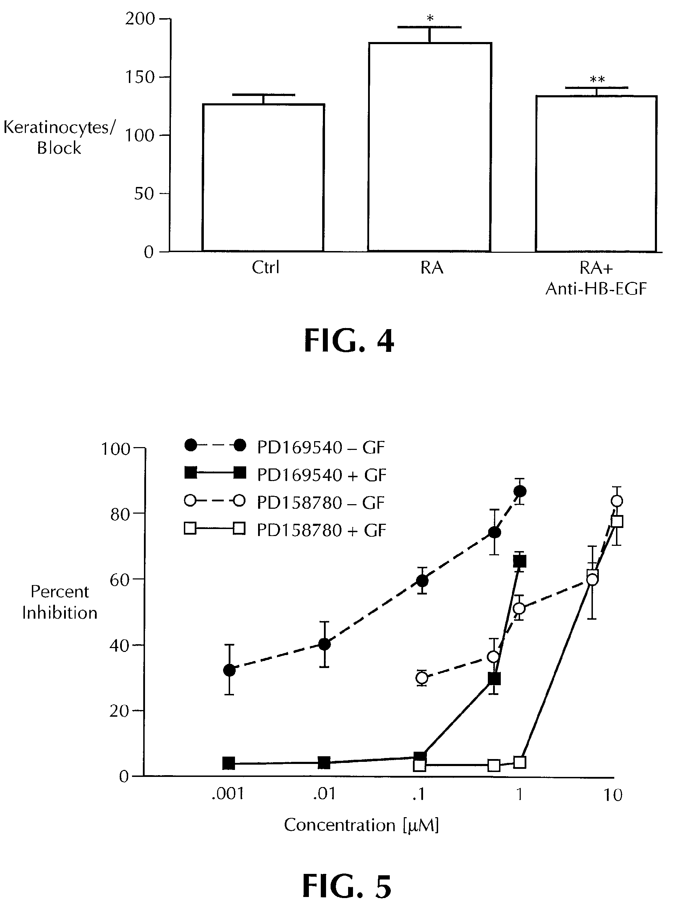

A goat polyclonal antibody to HB-EGF (cat. #AF-259-NA) was obtained from R&D Systems Inc. (Minneapolis, MN). The antibody neutralizes HB-EGF activity in Balb/3T3 cell proliferation assays. Normal goat serum was used as control. RA and ROL were obtained from Sigma Chemical Co. (St. Louis, MO). Stocks of RA were prepared at 20 mg/mL in dimethyl sulfoxide (DMSO) and stored frozen, protected from light. For *in vitro* assays, the RA stock was diluted in culture medium at the time of use. At the highest concentration of RA used (3 µM), the amount of DMSO in the culture medium was 0.05 µL/mL of culture medium. This amount of DMSO had no measurable effect on tissue structure or function (Varani J, Larson BK, Perone P, Inman DR, Fligiel SE, Voorhees JJ. All-trans rednoic acid and extracellular Ca2+ differentially influence extracellular matrix production by human skin in organ culture. *Am J Pathol,* 1993;142:1813-1822). A monoclonal antibody (mib-1) to the proliferation-associated antigen Ki-67 was obtained from Immunotech (Westbrook, ME).
Topical retinoid treatment of human skin: Adult volunteers were treated topically with RA, ROL, or vehicle on sun-protected buttock skin sites. The vehicle consisted of 7 parts 95% ethanol and 3 parts propylene glycol (v/v). Subjects were treated for 3 days with 0.1 % RA or vehicle, or for 7 days with 1% retinol (ROL) or vehicle. All treatments were applied under plastic wrap occlusion and covered with an opaque bandage to prevent drug loss and drug exposure to light. At the end of the treatment period, replicate 2-mm full-thickness punch biopsies of retinoid-treated and vehicle-treated skin were obtained from each individual. Tissue was fixed in 10% buffered formalin and examined histologically after staining with hematoxylin and eosin. The number of keratinocytes per unit length of epidermis was assessed morphometrically using NIH Image software as previously described (Varani J, Warner RL, Gharaee-Kermani M, Phan SH, Kang S, Chung JH, et al. Vitamin A antagonizes decreased cell growth and elevated collagen-degrading matrix metalloproteinases and stimulates collagen accumulation in naturally aged human skin. *J Invest Dermatol,* 2000; 114:480-486). Additional tissue sections were stained with antibody to Ki-67, and the number of positive cells counted per unit length of epidermis. Ki-67 expression is associated with proliferation (Key G, Becker MH, Baron B, Duchrow M, Schluter C, Flad HD, Gerdes J. New Ki-67-equivalent murine monoclonal antibodies (MIB 1-3) generated against bacterially expressed parts of the Ki-67 cDNA containing three 62 base pair repetitive elements encoding for the Ki-67 epitope. *Lab Invest,* 1993;68:629-636).
Human skin organ cultures: For the experiments shown in Figures 2 and 5, replicate 2 mm full-thickness punch biopsies of sun-protected hip skin were obtained from volunteers. Immediately upon biopsy, the tissue was immersed in culture medium consisting of Keratinocyte Basal Medium (KBM) (Clonetics, Inc., Walkersville. MD). KBM is a low-Ca²⁺, serum-free modification of MCDB-153 medium optimized for high-density keratinocyte growth (Wille J, Jr., Pittelkow MR, Shipley GD. Scott RE. Integrated control of growth and differentiation of normal human prokeratinocytes cultured in serum-free medium: clonal analyses, growth kinetics, and cell cycle studies. *J Cell Physiol* 1984;121:31-44). It was supplemented with CaCl₂ to bring the final Ca²⁺ concentration to 1.4 mM. After transport to the laboratory on ice, the biopsies were incubated in a 96-well dish containing 200 µL of Ca²⁺-supplemented KBM with or without additional treatments. Cultures were incubated at 37°C in an atmosphere of 95% air and 5% CO₂. Incubation was for 8 days, with change of medium containing the various treatments every 2 days. At the end of the incubation period, tissue was fixed in 10% buffered formalin and examined histologically after staining with hematoxylin and eosin or after staining with antibody to Ki-67. For the experiment shown in Figure 4, human skin keratome biopsies were subjected to organ culture in the presence or absence of 3 µM RA followed by RNA isolation and Northern blotting as previously described (Stoll SW, Elder JT. Retinoid regulation of heparin-binding EGF-lke growth factor gene expression in human keratinocytes and skin. *Exp Dermatol,* 1998;7:391-397), except that the concentration of Ca²⁺ was adjusted to 0.1 mM or 1.4 mM by the addition of CaCl₂. cDNA insert probes for detection of HB-EGF and 36B4 were prepared and labeled by random priming, as previously described (Stoll and Elder, Supra., 1998).
Human epidermal keratinocytes and dermal fibroblasts in monolayer culture: Normal human epidermal keratinocytes were obtained from skin biopsies and maintained in monolayer culture using Keratinocyte Growth Medium (KGM) (Clonetics, Inc., Walkersville, MD). KGM contains the same basal medium as KBM, but is further supplemented with a mixture of growth factors including 0.1 ng/mL EGF, 0.5 µg/mL insulin, and 2% bovine pituitary extract. In some experiments, the HaCaT line of immortalized human epidermal keratinocytes (Boukamp P, Petrussevska RT, Breitkreutz D, Hornung J, Markham A, Fusenig NE. Normal keratinization in a spontaneously immortalized aneuploid human keratinocyte cell line. *J Cell Biol,* 1988;106:761-771) was used in place of normal keratinocytes. We (J.V., S.W.S., and J.T.E., unpublished observations) and others (Chaturvedi V, Qin JZ, Denning MF, Choubey D, Diaz MO, Nickoloff BJ. Apoptosis in proliferating, senescent, and immortalized keratinocytes. *J Biol Chem,* 1999;274:23358-23367) have observed that HaCaT cells grow well in KGM. Therefore, HaCaT cells and NHK were propagated in exactly the same manner. Fibroblasts obtained from human skin were grown in monolayer culture using Dulbecco's modified minimal essential medium supplemented with nonessential amino acids and 10% fetal bovine serum (DMEM-FBS). Both keratinocytes and fibroblasts were maintained at 37°C in an atmosphere of 95% air and 5% CO₂. Cells were sub-cultured by exposure to trypsin/EDTA and used at passage 2-3.
Proliferation and cytotoxicity assays: For proliferation assays, cells were initially plated at 4 × 10⁴ cells per well of a 24-well dish in growth medium (KGM for keratinocytes or DMEM-FBS for fibroblasts). After allowing cells to attach for 1 to 2 hours, the medium was removed and the cells washed twice in KBM (keratinocytes) or Ca²⁺-supplemented KBM (fibroblasts). The cells were then incubated for 48 hours with or without additional reagents as indicated in Results. At the end of the incubation period, the cells were harvested by trypsinization and enumerated using an automated particle counter (Coulter Electronics, Hialeah, FL).

A ⁵¹Cr-release assay was used to measure cytotoxic effects of the irreversible c-erbB-RTK antagonist. For this assay, keratinocytes were initially plated at 2 × 10⁴ cells per well of a 24-well dish in growth medium (KGM) and treated with 1 µCi per well of ⁵¹Cr. After allowing cells to incorporate ⁵¹Cr for one day, the non-incorporated isotope was removed and the cells washed twice in KBM. The cells were then incubated for 18 hours with or without treatments as indicated in Results. At the end of the incubation period, the percentage of incorporated ⁵¹Cr that was released into the culture medium was determined. Our previous studies have demonstrated the relationship between ⁵¹Cr-release and lethal injury in human epidermal keratinocytes (Varani J, Inman DR, Perone P, Fligiel SE, Voorhees JJ. Retinoid toxicity for fibroblasts and epithelial cells is separable from growth promoting activity. *J Invest Dermatol* 1993;101:839-842).

### RESULTS

Induction of epidermal thickening in adult human skin by topical retinoid treatment: Comparison with hyperplasia induced in organ-cultured human skin by RA. As can be seen in Figure 1, topical application of RA or ROL under occlusion induced a marked increase in epidermal thickness, and in the number of keratinocytes present per linear unit of epidermis. It can also be seen that the number of Ki-67-positive cells increased markedly, in parallel with skin thickening, in the retinoid-treated groups.

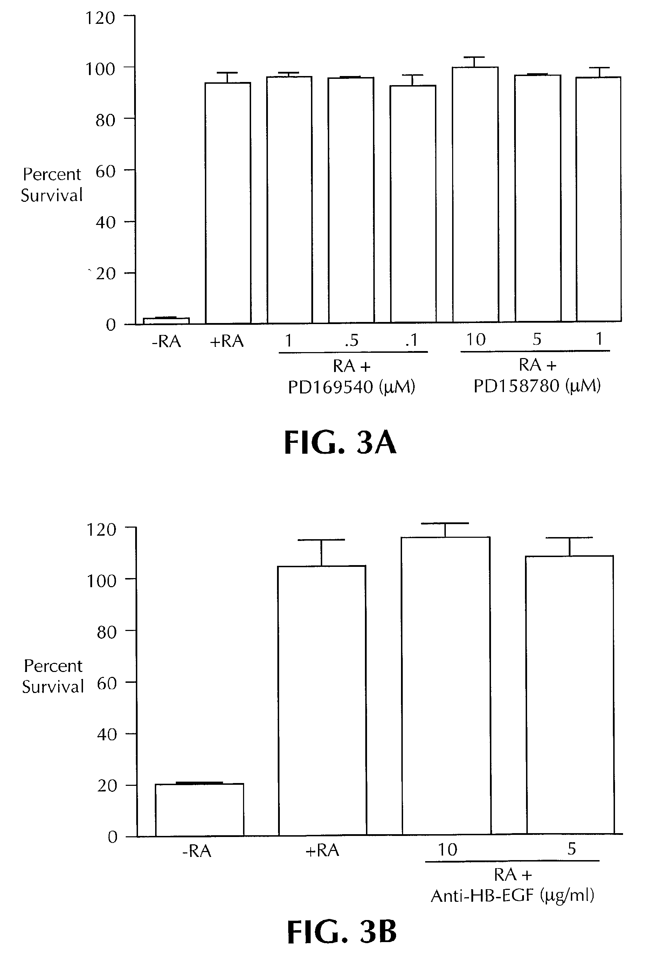

Adult human skin was maintained in organ culture for 8 days in the presence or absence of 3 µM RA. Figure 2 shows the typical histological appearance of organ-cultured tissue maintained in the absence or presence of RA. In control medium (KBM containing 1.4 mM Ca²⁺ but no RA), the histological appearance of the tissue resembled that of freshly-biopsied skin, as previously reported (Varani, Supra., 1993). Scattered cells in the basal layer stained positive with antibody to the proliferation-associated antigen, Ki-67, and occasional mitoses were observed (data not shown). The appearance of the RA-treated tissue was significantly different. The epidermis was thickened, due to an increase in the number of epidermal keratinocytes. Basal and lower suprabasal cells demonstrated a characteristic pallor on hematoxylin and eosin staining. The epidermis of RA-treated tissue was more disorganized than controls, with loss of the normal progression from basal to spinous to granular layers. Loss of suprabasal keratinocyte cohesion was frequently observed, leading to partial or complete separation of the upper spinous layers in some RA-treated specimens (data not shown). As was observed in retinoid-treated skin *in vivo* (Figure 1), RA-treated cultures displayed a much larger number of Ki-67-positive cells than did the controls, with Ki-67-positive cells in the basal and first suprabasal layers (not shown).
Effects of c-erbB-RTK antagonists on ability of RA to induce epidermal hyperplasia in organ-cultured skin. It can be seen that PD 169540 reversed the effects of 3 µM RA at the concentration used (1 µM). It has been established previously that this concentration of PD 169540 is without effect on tyrosine kinases residing outside the c-erbB family (Smaill JB, Rewcastle GW, Loo JA, Greis KD, Chan OH, Reyner EL. Tyrosine kinase inhibitors, Irreversible Inhibitors of the epidermal growth factor receptor: 4-(Phenylamino)quinazoline-and 4-(phenylamino)pyrido[3,2-d]pyrimidine-6-acrylamides bearing additional solubilizing functions. *J Med Chem,* 2000;43:1380-1397). The histological appearance of organ cultures treated with RA + PD 169540 was very similar to that of control organ cultures not treated with either reagent. Both the increase in epidermal thickness induced by RA and the diminution of RA-stimulated epidermal thickness by PD 169540 were statistically significant, as demonstrated by keratinocyte counts (Figure 2). The effect of PD 169540 was dose-dependent: 0.5 µM and 0.1 µM PD 169540 were also effective, albeit less so than 1 µM, whereas lower concentrations had no effect (data not shown). A reversible inhibitor of c-erbB-RTK activity, PD 158780, was examined at 1 µM, 5 µM, and 10 µM for effects on RA-stimulated hyperplasia. Like PD 169540, this compound also reversed the effects of RA. Antiproliferative activity was detected only at 5 and 10 µM PD 158780 (data not shown).
Effects of c-erbB-RTK antagonists on proliferation of human epidermal keratinocytes in monolayer culture. The two c-erbB-RTK antagonists were examined for their ability to inhibit the proliferation of human keratinocytes in monolayer culture (Figure 3). Both reagents inhibited keratinocyte growth in a concentration-dependent manner. There was a direct relationship between effectiveness in inhibiting hyperplasia in organ culture and effectiveness in blocking epithelial cell proliferation in monolayer culture. PD 169540 was clearly the more potent compound in both assays. Additional studies showed that both reagents were substantially more potent when the non-c-erbB keratinocyte mitogens, insulin and bovine pituitary extract (Wille et al, Supra., 1984; Boyce S, Ham R. Normal human epidermal keratinocytes. In: Webber M, Sekely L (ed). In Vitro Models for Cancer Research. CRC Press, Boca Raton, FL, 1983:245-274), were omitted from the keratinocyte cultures (Table 1). Essentially identical results were obtained with HaCaT cells as with low passage keratinocytes (data not shown). As assessed by ⁵¹Cr release assay, there was virtually no cytotoxicity of PD 169540 at any of the concentrations tested (Table 1).

**Table 1.**

| Growth Inhibition and Cytotoxicity of PD 169540 for Keratinocytes Under Various Conditions | | |
|---|---|---|
| Culture Conditions | Inhibition of Growth (ID₅₀, µRM)* | Cytotoxicity (ID₅₀, µM)** |
| KBM + Ca²⁺ | 0.008 | >1 |
| + EGF | 0.006 | >1 |
| + insulin/pituitary extract | 0.9 | >1 |

| | | |
|---|---|---|
| * Proliferation assays were carried out as described in the Materials and Methods section. Data are expressed as the dosage which inhibited growth by 50% based on two separate experiments. | | |
| ** Cytotoxicity assays were carried out as described in the Materials and Methods section. Data are expressed as the dosage which resulted in 50% of the cells killed, based on duplicate samples in a single experiment. | | |

Retinoid stimulation of HB-EGF mRNA in organ culture is intact under high-calcium conditions. Whereas all of the long-term organ culture studies presented here were carried out under high-Ca²⁺ conditions (1.4 mM), previous demonstrations that retinoids could potentiate HB-EGF expression in organ culture were carried out under low (0.1 mM) Ca²⁺ conditions (Stoll and Elder, Supra., 1998). In our previous studies we showed that elevation of the calcium concentration in the culture medium to 1.4 mM profoundly depressed the level of HB-EGF mRNA in organ culture (Xia L, Stoll SW, Liebert M, Ethier SP, Carey T, Esclamado R, et al. CaN19 expression in benign and malignant hyperplasias of the skin and oral mucosa: evidence for a role in regenerative differentiation. *Cancer Research* 1997:57:3055-3062). To determine whether RA could induce HB-EGF under high-Ca²⁺ conditions, organ cultures of adult human skin were incubated for 8 hours in basal medium containing either 0.1 mM or 1.4 mM Ca²⁺, in the presence or absence of 3 µM RA (Figure 4). Consistent with previous results, HB-EGF mRNA levels were strongly up-regulated in low-Ca²⁺ medium (Xia et al., Supra., 1997), and this induction was strongly potentiated by RA (Stoll and Elder, Supra., 1998). Moreover, HB-EGF induction was markedly diminished by elevation of the medium Ca²⁺ to 1.4 mM (Xia et al., Supra., 1997). In the presence of both high Ca²⁺ and RA, the stimulatory effect of 3 µM RA was clearly dominant over the inhibitory effect of 1.4 mM Ca²⁺, demonstrating that RA can induce HB-EGF under the calcium concentration required for long-term maintenance of organ cultures.
Effects of neutralizing anti-HB-EGF on RA-induced epidermal hyperplasia in organ-cultured skin. Organ cultures of adult human skin were treated for 8 days with 3 µM RA and concomitantly with 10 µg/mL of either a neutralizing antibody to HB-EGF or normal goat serum. Fresh culture media and reagents were added at 2-day intervals. At the end of the incubation period, the tissue was examined histologically. In the presence of antibody to HB-EGF, RA-stimulated hyperplasia was consistently inhibited (Figure 4). Again, the response was dose-dependent: 5 µg/mL anti-HB-EGF also inhibited RA-induced changes, albeit less effectively than 10 µg/mL. Concentrations lower than 5 µg/mL had no detectable effect (data not shown).
Effects of c-erbB-RTK antagonists and antibody to HB-EGF on RA-induced survival and proliferation of human dermal fibroblasts. After propagation in DMEM-FBS, human dermal fibroblasts were allowed to attach for 2 hours in DMEM-FBS, then propagated for 48 hours in KBM containing 0.15 mM CaCl₂ (low-Ca²⁺), with or without 3 µM RA. RA markedly and significantly promoted fibroblast survival under these low-Ca²⁺ conditions. There was no inhibition of RA-stimulated fibroblast survival by the c-erbB-RTK inhibitors or by anti-HB-EGF antibody under these conditions (Figure 5).

The foregoing data establish that survival of organ-cultured human skin requires a medium Ca²⁺ concentration that is optimal for fibroblast growth (e.g., 1.4 mM) rather than for growth of keratinocytes (e.g., 0.1 mM). Surprisingly, the addition of RA to low-Ca²⁺ cultures prevents epidermal degeneration, preserving epithelial viability for up to 12 days. RA-induced survival of skin in organ culture under low-Ca²⁺ conditions is mediated by effects on dermal elements since RA preserves fibroblast viability and promotes growth of these cells under the same low-Ca²⁺ conditions that preserve organ-cultured skin. The data clearly demonstrate that the effects of RA on dermal fibroblasts were not blocked by inhibitors of c-erbB-RTK activity or by a neutralizing antibody against HB-EGF. This finding stands in marked contrast to the pronounced effects of these reagents on cultured keratinocytes. Taken together, these findings clearly establish that while a viable dermis may be required for preservation of epidermal structure, and while retinoid effects on dermal cells may be required for preservation of organ-cultured skin under low-Ca²⁺ conditions, the hyperproliferative effects of retinoids are exerted directly upon keratinocytes, at least in part, via the action of HB-EGF on c-erbB receptors.

Clearly, while dermal and epidermal elements are present in both living skin *in vivo* and in organ culture *ex vivo,* these two settings differ in several important ways. Therefore, it is important to ask whether the molecular events causing retinoid hyperplasia in organ-cultured skin are the same as those producing retinoid hyperplasia in intact skin. Retinoid preparations that induce keratinocyte proliferation and epidermal thickening *in vivo* (0.025%-0.1% RA) produce drug concentration of 1 to 3 µM in the viable portion of the epidermis. This is the same concentration range that is effective in organ culture (Stoll and Elder, Supra., 1998, and this report), but it is higher by 10- to 100-fold, the concentration required for activation of retinoid receptors *in vitro.* Also, since Ca²⁺ concentrations are on the order of 1.4 mM in the extracellular fluid *in vivo,* the data presented in Figure 4 clearly establish that retinoids are able to overcome the effects of high calcium to induce HB-EGF after topical application *in vivo* as well as in organ culture. Taken together, these characteristics indicate that the hyperplastic response to retinoids proceeds via the same mechanism in organ culture and intact skin, and the foregoing data establish that addition of an erb inhibitor surprisingly reverses this undesirable retinoid activity.

Fibroblasts are the major cellular source of collagen production in the skin. Increased collagen production and reduced collagen degeneration are thought to be responsible for the antagonistic effect of topical retinoids on photoaging. Likewise, induction of new collagen elaboration may underlie the beneficial effects of topical retinoids in natural-aged skin. In spite of these beneficial effects, topical retinoids can induce undesirable effects such as scaling and redness in some people. The foregoing data show that c-erbB signaling appears to play little if any role in the effect of RA on fibroblasts. Accordingly, by administering an erb inhibitor to patient receiving a retinoid, it is now possible to dissociate the clinically undesirable effects of retinoids on epidermal keratinocytes from their desirable effects on dermal collagen biosynthesis and degradation. This invention thus provides compositions of retinoids and erb inhibitors and a method for treating skin disorders in mammals by administering such combinations, thereby achieving the benefits of retinoids on skin disorders without imposing the life-threatening detrimental toxicities often caused by retinoids alone.

Numerous compounds are known which are characterized as retinoids. A comprehensive discussion of retinoids is given by Dawson and Hobbs, in Chapter 2 of *The Retinoids: Biology, Chemistry, and Medicine,* 2^{nd} ed., Sporn, Roberts, and Goodman. New York: Raven Press, Ltd., 1994. That reference is incorporated herein by reference for its teaching of the synthesis of retinoids. All that is required by this invention is that a compound characterized as a retinoid is administered to an animal in an LF(a) lowering amount.

Preferred retinoids to be utilized in the present invention include retinoic acid of the formula

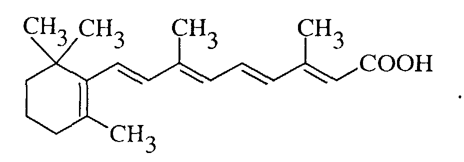

Retinoic acid derivatives also are preferred, for example, compounds of the formula 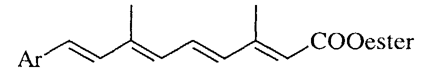 wherein Ar is an aryl group, and "ester" is an organic ester forming group.

Retinoids which are dienyl benzoic acid and enzynylaryl carboxylic acids also are preferred. For example, compounds of the formula 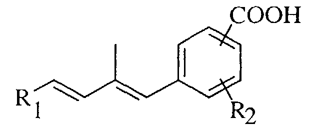 where R₁ is cycloalkyl or aryl, and R₂ is a typical phenyl substituted group such as halo, alkyl, alkoxy, alkylthio, and the like.

Compounds such as 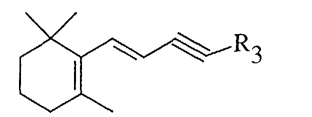 where R₃ is, for instance 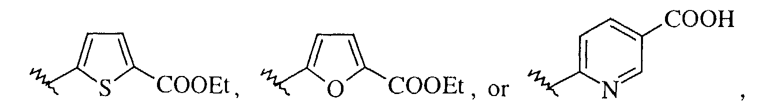 also are preferred.

Preferably, the retinoid is selected from *trans*-retinal, all*-trans* retinol, all-*trans*-retinoic acid, 9-*cis*-retinoic acid, 13-*cis*-retinoic acid, 13-*cis*-retinol, *13-cis*-retinal, 9-*cis*-retinal, or 9-*cis*-retinol.

All of the retinoids required for this invention are known and available by well-known synthetic methodologies.

## Claims

1. A composition of a retinoid and an erb inhibitor.

2. A composition according to Claim 1 where a retinoid is selected from all-*trans*-retinal, all*-trans* retinol, all*-trans* retinoic acid, 9-cis-retinoic acid, 13-cis-retinoic acid, 13-cis-retinal, 13-cis-retinol, 9-cis-retinal, or 9-cis-retinol.

3. A composition according to Claim 2 wherein the erb inhibitor is a quinazoline or a pyridopyrimidine; or
wherein the erb inhibitor is a quinazoline; or
the erb inhibitor is a pyridopyrimidine.

4. A composition according to Claim 3 where the erb inhibitor is a compound according to Formula II 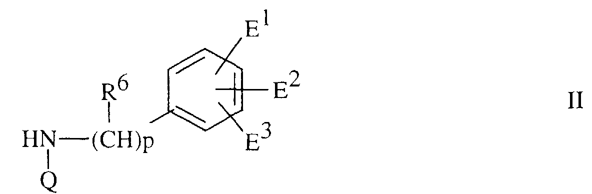 wherein
Q is 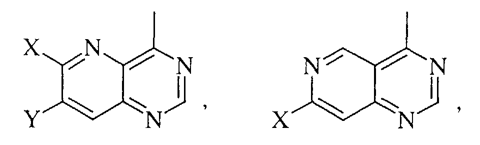 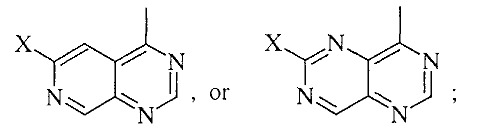
p is 0 or 1;
X is -D-E-F and Y is -SR⁴, -OR⁴, -NHR³, or hydrogen, or X is -SR⁴, -OR⁴, -NHR³, or hydrogen, and Y is -D-E-F;
D is 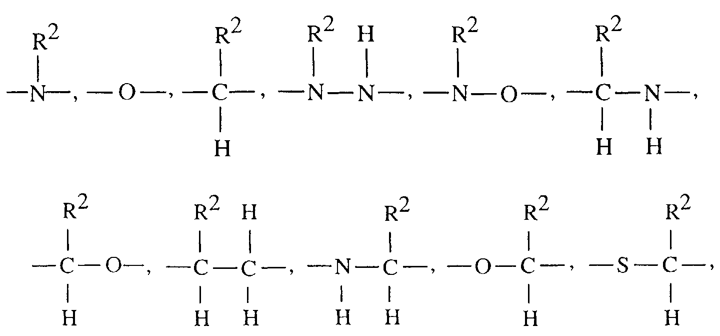 or absent;
E is 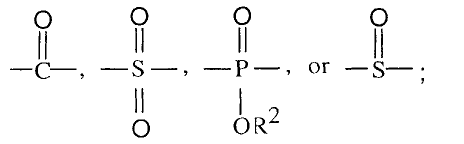
F is 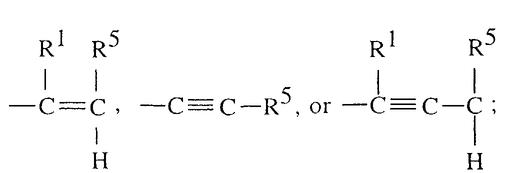 provided that when
E is 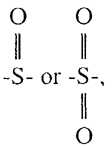
D is not 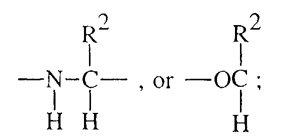
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from OH, -NH₂, or 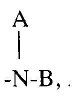 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
E¹, E², and E³ are independently halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C₆ alkyl, -(CH₂)ₙ-N-piperidinyl, -(CH₂)ₙ-piperazinyl, -(CH₂)ₙ-piperazinyl[N₄-(C₁-C₆)alkyl],-(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 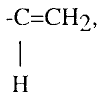 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆ alkyl)₂, -1-oxo(C₁-C₆)alkyl, carboxy, (C₁-C₆) alkyloxycarbonyl, N-(C₁-C₆)alkylcarbamoyl, phenyl or substituted phenyl, wherein the substituted phenyl can have from
one to three substituents independently selected from Z¹, Z², Z³ or a monocyclic heteroaryl group, and each C₁-C₆ alkyl group can be substituted with -OH, -NH₂ or -NAB, where A and B are as defined above, R⁶ is hydrogen or C₁-C₆ alkyl; and
n is 1 to 4, p is 0 and 1, and the pharmaceutically acceptable salts, esters, amides, and prodrugs thereof.

5. A composition according to Claim 4 wherein the erb inhibitor is 5-(4-methyl-piperazin-1-yl)-pent-2-ynoic acid [4-(3-chloro-4-fluorophenylamino)-pyrido[3,4-d]pyrimidin-6-yl]-amide; or
wherein the erb inhibitor is N⁴-(3-bromo-phenyl)-N⁶-methyl-pyrido[3,4-d]pyrimidine-4,6-diamine.

6. A composition according to Claim 3 wherein the quinazoline is a compound of Formula I 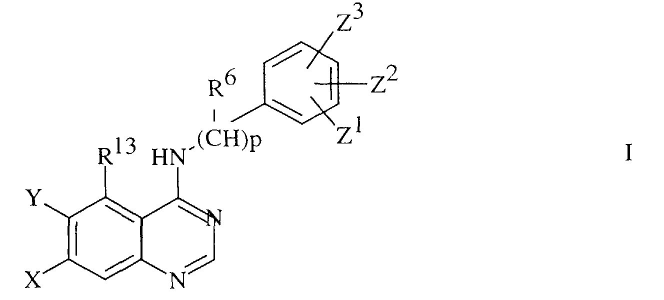 wherein
X is -D-E-F and Y is -SR⁴, halogen, -OR⁴, -NHR³, or hydrogen, or X is -SR⁴, halogen, -OR⁴, -NHR³, or hydrogen, and Y is -D-E-F;
D is 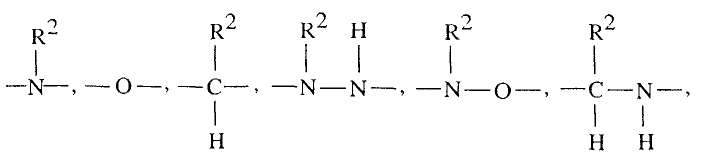 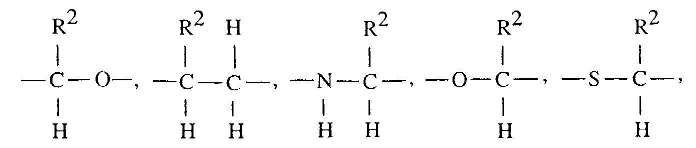 or absent;
E is 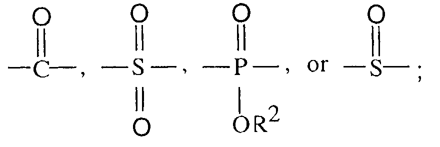
F is 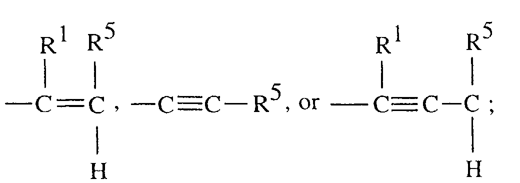 provided that when
E is 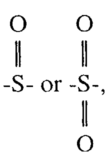
D is not 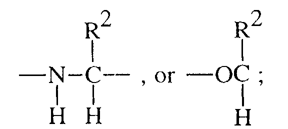
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from -OH, -NH₂, or 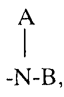
A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆-)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
Z¹, Z², or Z³ are independently hydrogen, halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C₆alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 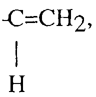 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆alkyl)₂, -1-oxo(C₁-C₆)alkyl, carboxy, (C₁-C₆)alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group above in R⁵ can be substituted with -OH, -NH₂ or -NAB, where A and B are as defined above, R⁶ is hydrogen or C₁-C₆ alkyl; R¹³ is hydrogen or halogen; and
n is 1 to 4, p is 0 or 1, and the pharmaceutically acceptable salts, esters, amides, and prodrugs thereof.

7. A composition of Claim 6 wherein the quinazoline is a 4-phenyl or substituted phenylamino compound; or
wherein the erb inhibitor is N-[4-(3-chloro-4-fluoro-phenylamino)-7-(3-morpholin-4-yl-propoxy)-quinazolin-6-yl]-acrylamide or a salt thereof; or
wherein the erb inhibitor is N-[4-(3-bromo-phenylamino)-7-(3-morpholin-4-yl-propoxy)-quinazolin-6-yl]-acrylamide.

8. A kit containing a retinoid in one compartment and an erb inhibitor in a second compartment; or
wherein a retinoid is selected from all-*trans*-retinal, all*-trans* retinol, all-*trans* retinoic acid, 9-cis-retinoic acid, 13-cis-retinoic acid, 13-cis-retinal, 13-cis-retinol, 9-cis-retinal, or 9-cis-retinol; or
wherein the erb inhibitor is a pyridopyrimidine; or
wherein the erb inhibitor is a compound according to Formula II 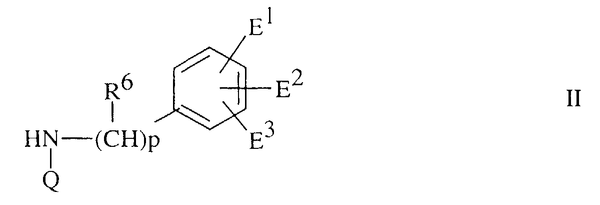 wherein
Q is 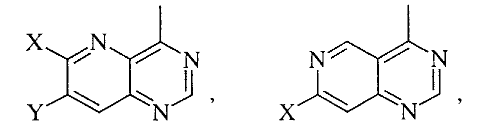 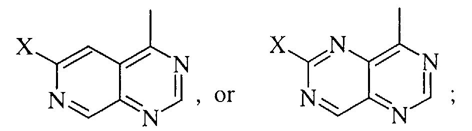
p is 0 or 1;
X is -D-E-F and Y is -SR⁴, -OR⁴, -NHR³, or hydrogen, or X is -SR⁴, -OR⁴, -NHR³, or hydrogen, and Y is -D-E-F;
D is 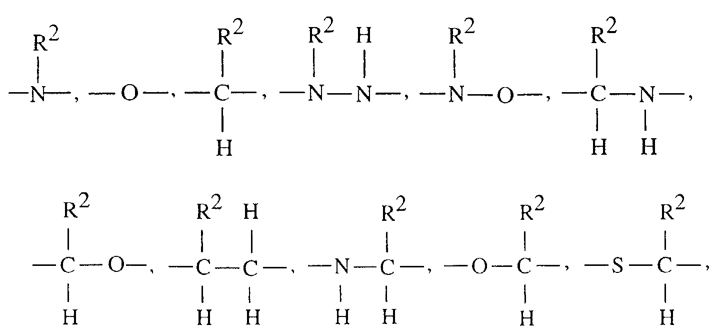 or absent;
E is 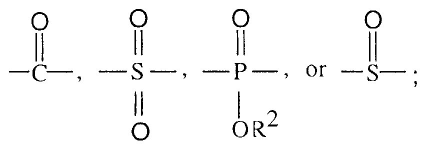
F is 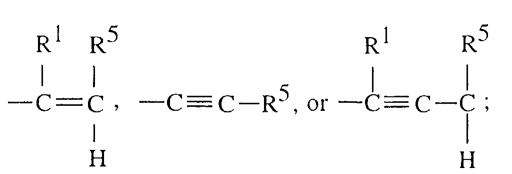 provided that when
E is 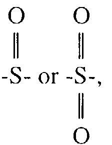 D is not 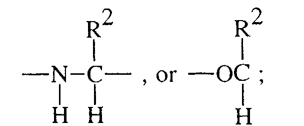
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from OH, -NH₂, or 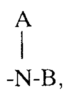 A and B are independently hydrogen, C₁-C₆ alkyl, -(CH₂)ₙOH, - (CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
E¹, E², and E³ are independently halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C6 alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 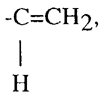 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆ alkyl)₂, -1-oxo(C₁-C₆)alkyl, carboxy, (C₁-C₆) alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group can be substituted with -OH, -NH₂ or -NAB, where A and B are as defined above, R⁶ is hydrogen or C₁-C₆ alkyl; and
n is 1 to 4, p is 0 and 1, and the pharmaceutically acceptable salts, esters, amides, and prodrugs thereof; or
wherein the erb inhibitor is 5-(4-methyl-piperazin-1-yl)-pent-2-ynoic acid [4-(3-chloro-4-fluoro-phenylamino)-pyrido[3,4-d]pyrimidin-6-yl]-amide; or
wherein the erb inhibitor is N⁴-(3-bromo-phenyl)-N⁶-methyl-pyrido[3,4-d]pyrimidine-4,6-diamine; or
wherein the erb inhibitor is a quinazoline; or
wherein the quinazoline is a compound of Formula I 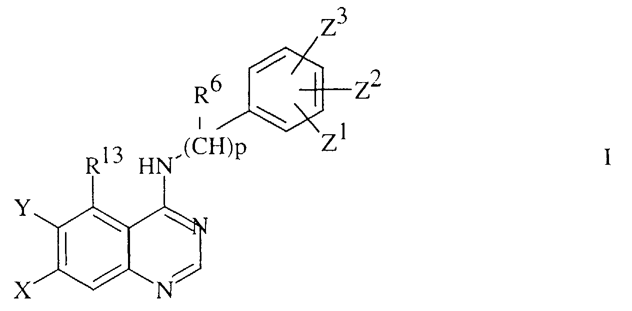 wherein
X is -D-E-F and Y is -SR⁴, halogen, -OR⁴, -NHR³, or hydrogen, or X is -SR⁴, halogen, -OR⁴, -NHR³, or hydrogen, and Y is -D-E-F:
D is 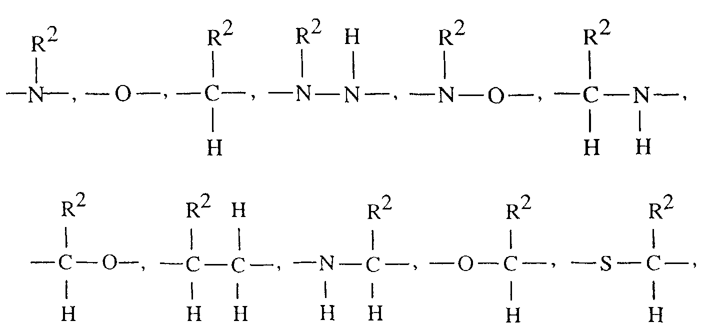 or absent;
E is 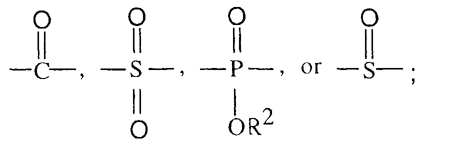
F is 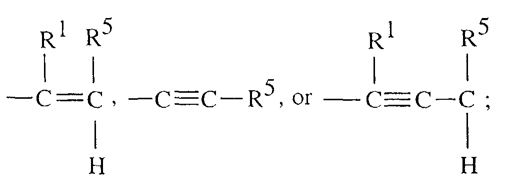 provided that when
E is 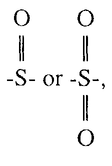
D is not 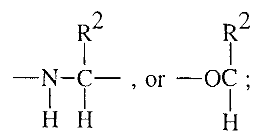
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -((CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from -OH, -NH₂, or 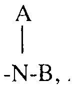 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆-)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
Z¹, Z², or Z³ are independently hydrogen, halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C₆alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 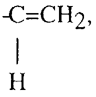 -CH=CH-(C₁-C₆)alkyl, -(CH₂)ₙ-N-hexahydroazepine, -(CH₂)ₙNH₂, -(CH₂)ₙNH(C₁-C₆alkyl),-(CH₂)ₙN(C₁-C₆alkyl)₂, -1-oxo(C₁-C₆)alkyl, carboxy, (C₁-C₆)alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group above in R⁵ can be substituted with -OH, -NH₂ or -NAB, where A and B are as defined above, R⁶ is hydrogen or C₁-C₆ alkyl; R¹³ is hydrogen or halogen; and
n is 1 to 4, p is 0 or 1, and the pharmaceutically acceptable salts, esters, amides, and prodrugs thereof; or
wherein the erb inhibitor is N-[4-(3-chloro-4-fluoro-phenylamino)-7-(3-morpholin-4-yl-propoxy)-quinazolin-6-yl]-acrylamide or a salt thereof; or
wherein the erb inhibitor is N-[4-(3-bromo-phenylamino)-7-(3-morpholin-4-yl-propoxy)-quinazolin-6-yl]-acrylamide.

9. The use of an antiskin disorder amount of an erb inhibitor in combination with a retinoid for the preparation of a pharmaceutical composition for treating and preventing retinoid-induced skin injury responsive to erb inhibition.

10. The use according to Claim 9 wherein a retinoid is selected from all-*trans*-retinal, all*-trans* retinol, all-*trans* retinoic acid, 9-cis-retinoic acid, 13-cis-retinoic acid, 13-cis-retinal, 13-cis-retinol, 9-cis-retinal, or 9-cis-retinol; or
wherein the erb inhibitor is a pyridopyrimidine; or
wherein the erb inhibitor is a compound according to Formula II 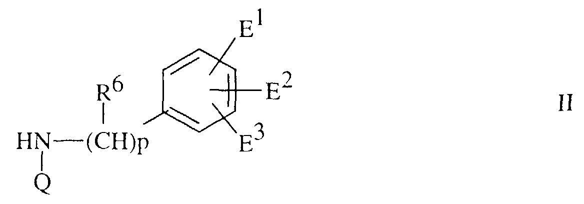 wherein
Q is 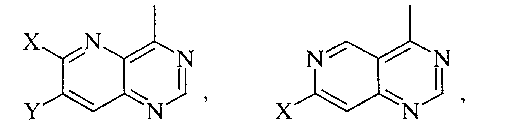 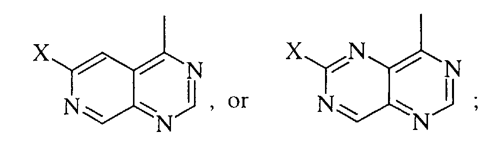
p is 0 or 1;
X is -D-E-F and Y is -SR⁴, -OR⁴, -NHR³, or hydrogen, or X is -SR⁴, -OR⁴, -NHR³, or hydrogen, and Y is -D-E-F;
D is 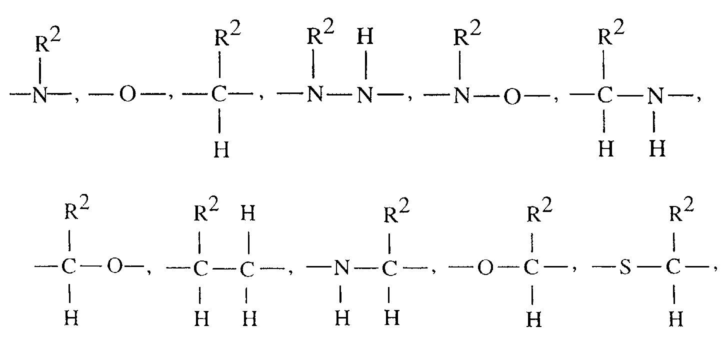 or absent;
E is 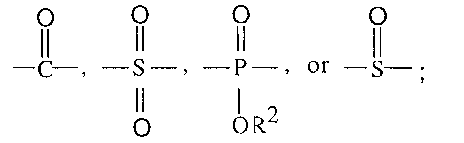
F is 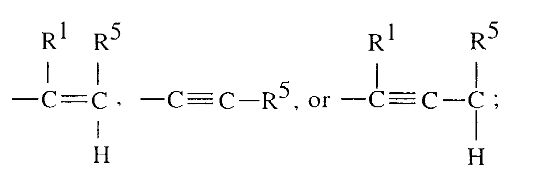 provided that when
E is 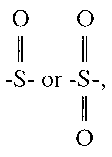
D is not x 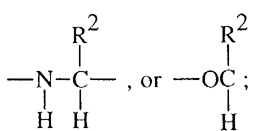
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C ₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from OH, -NH₂, or 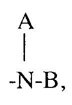 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
E¹, E², and E³ are independently halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C6 alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 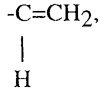 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆ alkyl)₂, -1-oxo(C₁-C₆)alkyl, carboxy, (C₁-C₆) alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group can be substituted with -OH, -NH₂ or -NAB, where A and B are as defined above, R⁶ is hydrogen or C₁-C₆ alkyl; and
n is 1 to 4, p is 0 and 1, and the pharmaceutically acceptable salts, esters, amides, and prodrugs thereof; or
wherein the erb inhibitor is 5-(4-methyl-piperazin-1-yl)-pent-2-ynoic acid [4-(3-chloro-4-fluoro-phenylamino)-pyrido[3,4-d]pyrimidin-6-yl]-amide; or
wherein the erb inhibitor is N⁴-(3-bromo-phenyl)-N⁶-methyl-pyrido[3,4-d]pyrimidine-4,6-diamine; or
wherein the erb inhibitor is a quinazoline; or
wherein the quinazoline is a compound of Formula I 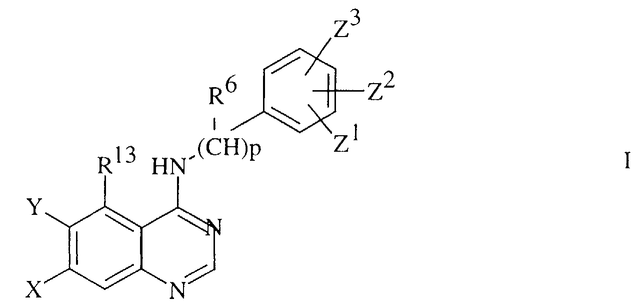 wherein
X is -D-E-F and Y is -SR⁴, halogen, -OR⁴, -NHR³, or hydrogen, or X is -SR⁴, halogen, -OR⁴, -NHR³, or hydrogen, and Y is -D-E-F;
D is 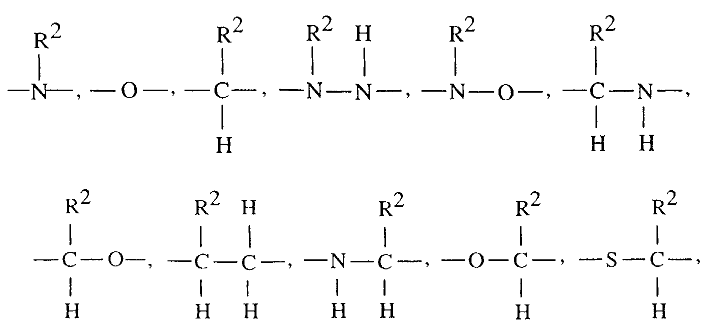 or absent;
E is 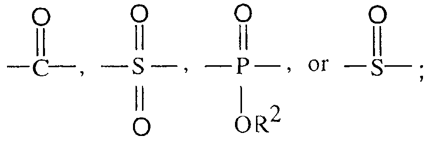
F is 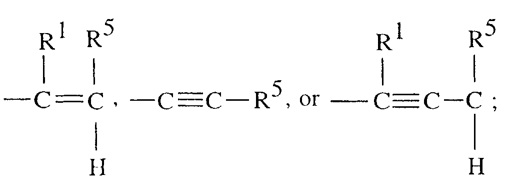 provided that when
E is 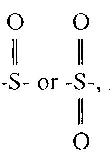
D is not 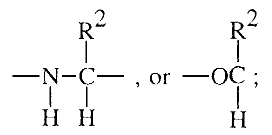
R¹ is hydrogen, halogen, or C₁-C₆ alkyl;
R², R³, and R⁴ are independently hydrogen, C₁-C₆ alkyl, -(CH₂)ₙ-N-piperidinyl, -(CH₂)ₙ-N-piperazinyl, -(CH₂)ₙ-N₁-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-imidazoyl, -(CH₂)ₙ-N-morpholino, -(CH₂)ₙ-N-thiomorpholino, -(CH₂)ₙ-N-hexahydroazepine or substituted C₁-C₆ alkyl, wherein the substituents are selected from -OH, -NH₂, or 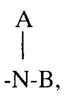 A and B are independently hydrogen, C₁-C₆ alkyl, -(CH₂)ₙOH, -(CH₂)ₙ-N-piperidinyl, -(CH₂)ₙ-N-piperazinyl, -(CH₂)ₙ-N₁-piperazinyl[N₄-(C₁-C₆-)alkyl], -(CH₂)ₙ-N-pyrrolidyl, -(CH₂)ₙ-N-pyridyl, -(CH₂)ₙ-imidazoyl, or -(CH₂)ₙ-N-imidazoyl;
Z¹, Z², or Z³ are independently hydrogen, halogen, C₁-C₆ alkyl, C₃-C₈ cycloalkyl, C₁-C₆ alkoxy, C₃-C₈ cycloalkoxy, nitro, C₁-C₆ perfluoroalkyl, hydroxy, C₁-C₆ acyloxy, -NH₂, -NH(C₁-C₆ alkyl), -N(C₁-C₆ alkyl)₂, -NH(C₃-C₈ cycloalkyl), -N(C₃-C₈ cycloalkyl)₂, hydroxymethyl, C₁-C₆ acyl, cyano, azido, C₁-C₆ thioalkyl, C₁-C₆ sulfinylalkyl, C₁-C₆ sulfonylalkyl, C₃-C₈ thiocycloalkyl, C₃-C₈ sulfinylcycloalkyl, C₃-C₈ sulfonylcycloalkyl, mercapto, C₁-C₆ alkoxycarbonyl, C₃-C₈ cycloalkoxycarbonyl, C₂-C₄ alkenyl, C₄-C₈ cycloalkenyl, or C₂-C₄ alkynyl;
R⁵ is hydrogen, halogen, C₁-C₆-perfluoroalkyl, 1,1-difluoro(C₁-C₆)alkyl, C₁-C₆alkyl, -(CH₂)ₙ-N-piperidinyl, -(CH₂)ₙ-piperazinyl, -(CH₂)ₙ-piperazinyl[N₄-(C₁-C₆)alkyl], -(CH₂)ₙ-N-pyrrolidyl, -(CH₂)ₙ-pyridinyl, -(CH₂)ₙ-N-imidazoyl, -(CH₂)ₙ-N-morpholino, -(CH₂)ₙ-N-thiomorpholino, 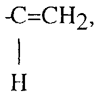 -CH=CH-(C₁-C₆)alkyl, -(CH₂)ₙ-N-hexahydroazepine, -(CH₂)ₙNH₂, -(CH₂)ₙNH(C₁-C₆alkyl), -(CH₂)ₙN(C₁-C₆alkyl)₂, -1-oxo(C₁-C₆)alkyl, carboxy, (C₁-C₆)alkyloxycarbonyl, N-(C₁-C₆)alkylcarbamoyl, phenyl or substituted phenyl, wherein the substituted phenyl can have from one to three substituents independently selected from Z¹, Z², Z³ or a monocyclic heteroaryl group, and each C₁-C₆ alkyl group above in R⁵ can be substituted with -OH, -NH₂ or -NAB, where A and B are as defined above, R⁶ is hydrogen or C₁-C₆ alkyl; R¹³ is hydrogen or halogen; and
n is 1 to 4, p is 0 or 1, and the pharmaceutically acceptable salts, esters, amides, and prodrugs thereof; or
wherein the erb inhibitor is N-[4-(3-chloro-4-fluoro-phenylamino)-7-(3-morpholin-4-yl-propoxy)-quinazolin-6-yl]-acrylamide dihydrochloride; or
wherein the erb inhibitor is N-[4-(3-bromo-phenylamino)-7-(3-morpholin-4-yl-propoxy)-quinazolin-6-yl]-acrylamide; or
wherein the erb inhibitor is N-[4-(3-chloro-4-fluoro-phenylamino)-7-(3-morpholin-4-yl-propoxy)-quinazolin-6-yl]-acrylamide.

11. The use according to Claim 10 wherein the skin disorder is aging; or
wherein the skin disorder is photoaging; or
wherein the skin disorder is acne; or
wherein the skin disorder is psoriasis; or
wherein the skin disorder is precancerous lesions of the skin; or
wherein the skin disorder is skin cancer.
